# EUROPEAN PATENT SPECIFICATION

(11) **EP 4 461 369 B1**
(45) Date of publication and mention of the grant of the patent: **25.02.2026**
(21) Application number: 24188510.2
(22) Date of filing: 15.04.2020
(51) Int. Cl.: A61K 9/72, A61K 35/12, A61K 35/33, A61K 35/34, A61P 11/00, A61P 35/00

(54) **AEROSOLIZED COMPOSITIONS COMPRISING MITOCHONDRIA AND METHODS OF USE THEREOF**
AEROSOLIERTE ZUSAMMENSETZUNGEN MIT MITOCHONDRIEN UND VERFAHREN ZU DEREN VERWENDUNG
COMPOSITIONS EN AÉROSOL COMPRENANT DES MITOCHONDRIES ET LEURS MÉTHODES D'UTILISATION

(30) Priority: 15.04.2019 US 201962834020 P
(43) Date of publication of application: 13.11.2024
(62) Divisional of application: 20790601.7
(73) Proprietor: Children's Medical Center Corporation, Boston, MA 02115 (US)
(72) Inventor: MCCULLY, James D., Marblehead, MA 01945 (US); DEL NIDO, Pedro J., Lexington, MA 02420 (US)
(74) Representative: Fish & Richardson P.C.

(56) References cited:
- EP-A1- 3 402 490
- SU Y. ET AL.: "Mitochondrial Transplantation Attenuates Airway Hyperresponsiveness by Inhibition of Cholinergic Hyperactivity", vol. 6, no. 8, 1 January 2016 (2016-01-01), AU, pages 1244 - 1260, XP055978572, ISSN: 1838-7640, Retrieved from the Internet <URL:https://www.thno.org/v06p1244.pdf> DOI: 10.7150/thno.13804

## Description

### TECHNICAL FIELD

This disclosure pertains to aerosolized compositions containing mitochondria, methods of preparing and using the compositions, and devices for administering the compositions.

### BACKGROUND

Mitochondria are double membrane-bound organelles found in the cytoplasm of nucleated eukaryotic cells. They are found in almost every cell of the human body except red blood cells. They are the cell's primary site of energy metabolism and generate adenosine triphosphate (ATP) for different cell functions. Typically, more than 90% of a cell's requirement for ATP is supplied by the cell's own mitochondria.

Mitochondria are composed of two concentric membranes, which have specialized functions. The inner mitochondrial membrane contains proteins for ATP synthase. The outer mitochondrial membrane, which contains large numbers of integral membrane proteins, encloses the entire organelle.

The structure of mitochondria has striking similarities to some modem prokaryotes. In fact, mitochondria are thought to have originated from an ancient symbiosis when a nucleated cell engulfed an aerobic prokaryote. In the symbiosis relationship, the host cell came to rely on the engulfed prokaryote for energy production, and the prokaryote cell began to rely on the protective environment provided by the host cell.

Due to mitochondria's primary function in cell metabolism, damage and dysfunction in mitochondria can cause a range of human diseases. Damage to mitochondria may be caused by injury, toxicity, chemotherapy, and age-related changes. Particularly, ischemia/reperfusion injury can cause mitochondrial damage, which will have a negative impact on oxygen consumption and energy synthesis. Treatments involving mitochondria can be particularly useful for mitochondria-related disorders. Some early treatments involve administering mitochondria by intra-muscular injection and intra-arterial injection. There is a need for administering mitochondria to various target sites of a subject through different administration routes for various purposes.

### SUMMARY

This disclosure provides compositions (e.g., aerosolized compositions) containing mitochondria, methods of preparing and using the compositions, and devices for administering the compositions. In one aspect, the disclosure provides methods of administering a composition containing mitochondria or combined mitochondrial agents through respiratory tract. In some embodiments, the composition is an aerosolized composition. In some embodiments, the composition is a liquid solution.

In one aspect, the disclosure relates to a method of treating a subject having a respiratory disorder, the method comprising administering a composition comprising a therapeutically effective amount of mitochondria to the subject through respiratory tract.

In some embodiments, the composition is an aerosolized composition. In some embodiments, the composition is converted into an aerosol form prior to administration to the subject by using a nebulizer, a vaporizer, a nasal sprayer, a pressurized metered dose inhaler, or a breath activated pressurized metered dose inhaler.

In some embodiments, the aerosol form of the composition comprises droplets that have a median size from 0.01 to 1000 microliters (e.g., from 0.1 to 1000 microliters, from 1 to 1000 microliters, from 0.1 to 100 microliters, from 0.1 to 500 microliters, or from 1 to 500 microliters). In some embodiments, the droplet has a size at least 0.01, 0.1, 0.5, 1, 2, 3, 4, 5, 6, 7, 8, 9, 10, 20, 30, 40, 50, 60, 70, 80, 90, 100, 200, 300, 400, 500, 600, 700, 800, 900, or 1000 microliters. In some embodiments, the droplet has a size less than 1, 2, 3, 4, 5, 6, 7, 8, 9, 10, 20, 30, 40, 50, 60, 70, 80, 90, 100, 200, 300, 400, 500, 600, 700, 800, 900, or 1000 microliters.

In some embodiments, the composition comprises respiration-competent mitochondria.

In some embodiments, the subject has respiratory failure, reduced respiratory function, lung inflammation, lung carcinoma, skin wrinkles, baldness, and/or cancer. In some embodiments, the subject has acute lung injury.

In some embodiments, the concentration of mitochondria in the composition is about 1x10⁴ to 5x10⁹ ml⁻¹ (e.g., about 1x10⁵ to 5x10⁸ ml⁻¹, 1x10⁶ to 5x10⁸ ml⁻¹, 1x10⁷ to 5x10⁸ ml⁻¹, 1x10⁵ to 1x10⁸ ml⁻¹, 1x10⁶ to 1x10⁸ ml⁻¹, 1x10⁷ to 1x10⁸ ml⁻¹, 1x10⁶ to 5x10⁷ ml⁻¹, 1x10⁶ to 1x10⁷ ml⁻¹, 1x10⁶ to 5x10⁷ ml⁻¹, 5x10⁶ to 1x10⁸ ml⁻¹). In some embodiments, the concentration is at least or about 1x10⁴ ml⁻¹, 5x10⁴ ml⁻¹, 1x10⁵ ml⁻¹, 5x10⁵ ml⁻¹, 1x10⁶ ml⁻¹, 5x10⁶ ml⁻¹, 1x10⁷ ml⁻¹, 5x10⁷ ml⁻¹, 1x10⁸ ml⁻¹, 5x10⁸ ml⁻¹, or 1x10⁹ ml⁻¹, 5x10⁹ ml⁻¹, or 1x10¹⁰ ml⁻¹. In some embodiments, the concentration is less than 1x10⁴ ml⁻¹, 5x10⁴ ml⁻¹, 1x10⁵ ml⁻¹, 5x10⁵ ml⁻¹, 1x10⁶ ml⁻¹, 5x10⁶ ml⁻¹, 1x10⁷ ml⁻¹, 5x10⁷ ml⁻¹, 1x10⁸ ml⁻¹, 5x10⁸ ml⁻¹, or 1x10⁹ ml⁻¹, 5x10⁹ ml⁻¹, or 1x10¹⁰ ml⁻¹.

In some embodiments, the subject is administered 1x10⁵ to 1x10⁹ of mitochondria per dose (e.g., 1x10⁵ to 5x10⁸, 1x10⁶ to 5x10⁸, 1x10⁷ to 5x10⁸, 1x10⁵ to 1x10⁸, 1x10⁶ to 1x10⁸, 1x10⁷ to 1x10⁸, 1x10⁶ to 5x10⁷, 1x10⁶ to 1x10⁷, 1x10⁶ to 5x10⁷, 5x10⁶ to 1x10⁸ mitochondria per dose). In some embodiments, the dose is at least 1x10⁴, 5x10⁴, 1x10⁵, 5x10⁵, 1x10⁶, 5x10⁶, 1x10⁷, 5x10⁷, 1x10⁸, 5x10⁸, or 1x10⁹, 5x10⁹, or 1x10¹⁰ mitochondria per dose. In some embodiments, the dose is less than 1x10⁴, 5x10⁴, 1x10⁵, 5x10⁵, 1x10⁶, 5x10⁶, 1x10⁷, 5x10⁷, 1x10⁸, 5x10⁸, or 1x10⁹, 5x10⁹, or 1x10¹⁰ mitochondria per dose.

In some embodiments, the mitochondria are autogenic, allogeneic, or xenogeneic.

In some embodiments, the composition further comprises a solution selected from the group consisting of: K⁺-HEPES with a pH from 7 to 8, saline, phosphate-buffered saline (PBS), serum, and plasma.

In some embodiments, the composition further comprises one or more osmolytes selected from the group consisting of: trehalose, sucrose, mannose, glycine, proline, glycerol, mannitol, sorbitol, betaine, and sarcosine.

In some embodiments, the composition further comprises a pharmaceutical agent. In some embodiments, the composition further comprises a pharmaceutically acceptable diluent, excipient, or carrier.

In some embodiments, the composition further comprises a therapeutic agent or a diagnostic agent, wherein the therapeutic agent or the diagnostic agent is linked to the mitochondria by a covalent bond, is embedded in the mitochondria, or is internalized within the mitochondria. In some embodiments, the therapeutic agent or the diagnostic agent is a therapeutic diagnostic agent. In some embodiments, the therapeutic agent or the diagnostic agent comprises an antibody or an antigen binding fragment thereof. In some embodiments, the therapeutic agent or the diagnostic agent is linked to the mitochondria by a covalent bond.

In some embodiments, the therapeutic agent or the diagnostic agent is embedded in or internalized within the mitochondria.

In some embodiments, the mitochondria are genetically modified.

In some embodiments, the mitochondria comprise exogenous polypeptides.

In some embodiments, the mitochondria comprise exogenous polynucleotides, DNA, RNA, mRNA, micro RNAs, nuclear RNAs, or siRNA.

In one aspect, the disclosure relates to an aerosolized composition comprising a plurality of liquid droplets comprising a buffer, wherein at least one liquid droplet in the plurality of liquid droplets comprises at least one mitochondrion.

In some embodiments, the buffer is selected from the group consisting of: K+-HEPES with a pH from 7 to 8, saline, phosphate-buffered saline (PBS), serum, and plasma.

In some embodiments, the composition further comprises one or more osmolytes selected from the group consisting of: trehalose, sucrose, mannose, glycine, proline, glycerol, mannitol, sorbitol, betaine, and sarcosine.

In some embodiments, the composition further comprises a pharmaceutical agent.

In some embodiments, the composition further comprises a pharmaceutically acceptable diluent, excipient, or carrier.

In some embodiments, the composition further comprises a therapeutic agent or a diagnostic agent, wherein the therapeutic agent or the diagnostic agent is linked to the mitochondrion by a covalent bond, is embedded in the mitochondrion, or is internalized within the mitochondrion.

In some embodiments, the mitochondrial agent is selected from the group consisting of a therapeutic agent, a chemotherapeutic agent, or a diagnostic agent.

In some embodiments, the mitochondrial agent comprises an antibody or an antigen binding fragment.

In some embodiments, the mitochondrial agent is linked to the mitochondrion by a covalent bond. In some embodiments, the mitochondrial agent is embedded in or internalized within the mitochondrion.

In some embodiments, the plurality of droplets have a median size from 1 to 1000 microliters.

In some embodiments, the mitochondrion are genetically modified.

In some embodiments, the mitochondrion comprise exogenous polypeptides.

In some embodiments, the mitochondrion comprise exogenous polynucleotides.

In some embodiments, the mitochondrion prior to administration to the subject are incubated with a composition comprising an enzyme.

In some embodiments, the composition comprises autogenic mitochondria, allogeneic mitochondria, xenogeneic mitochondria, or a mixture thereof.

In one aspect, the disclosure relates to a device for delivering mitochondria to a subject through respiratory tract, the device comprising: a housing; a reservoir disposed within the housing for a composition comprising mitochondria; an aerosol generator to produce an aerosol form of the composition; and an outlet through which the composition is delivered to the respiratory tract of the subject.

In some embodiments, the device is a nebulizer, a nasal sprayer, or an inhaler. In some embodiments, the aerosol generator is an ultrasonic nebulizer.

In some embodiments, the aerosol generator is a vibrating mesh device. In some embodiments, the aerosol generator comprises an air compressor, wherein the air compressor cause compressed air to flow through the composition and turn the composition into an aerosol form.

In one aspect, the disclosure provides device for delivering mitochondria to a subject through respiratory tract, the device comprising: a housing; a composition comprising mitochondria disposed within the housing; and an outlet configured to deliver the composition from the housing to the respiratory tract of the subject and to aerosolize the composition as the composition passes therethrough.

Unless otherwise defined, all technical and scientific terms used herein have the same meaning as commonly understood by one of ordinary skill in the art to which this invention belongs. Methods and materials are described herein for use in the present invention; other, suitable methods and materials known in the art can also be used. The materials, methods, and examples are illustrative only and not intended to be limiting.

Other features and advantages of the invention will be apparent from the following detailed description and figures, and from the claims.

### BRIEF DESCRIPTION OF THE DRAWINGS

**FIG. 1** is a schematic diagram showing a method of isolating mitochondria.
**FIG. 2A** is skeletal muscle tissue that was obtained using a biopsy punch.
**FIG. 2B** are microscopic images of isolated mitochondria under phase contrast illumination (bright field, BF) (left panel), under fluorescence labeled with MitoTracker Red CMXRos (middle panel), and the merged image (the right panel).
**FIG. 2C** is a plot that shows results for mitochondria yield per gram tissue wet weight.
**FIG. 2D** are transmission electron microscopy images of isolated mitochondria.
**FIG. 2E** is a bar graph that shows results of activity assays for the mitochondrial complex I-V.
**FIG. 2F** is a bar graph that shows the results for the state 3 (active) oxygen consumption (ADP-stimulated respiration).
**FIG. 2G** is a bar graph that shows results for the respiratory control index (RCI; state 3/state 4) for malate induced (complex I) and succinate induced (complex II) oxygen consumption in autologous mitochondria from pectoralis major.
**FIG. 3A** are overlaid microscope images showing transplanted mitochondria localized within cardiomyocytes.
**FIG. 3B** are overlaid microscope images showing transplanted mitochondria localized within cardiomyocytes.
**FIG. 3C** are overlaid microscope images showing transplanted mitochondria localized within cardiomyocytes
**FIG. 3D** are overlaid microscope images showing transplanted mitochondria localized within cardiomyocytes
**FIG. 4A** is an image that shows a representative transverse image obtained by positron emission tomography (PET) of rats administered ¹⁸F Rhodamine 6-G mitochondria (6 x 10⁷) delivered to the lungs by vascular infusion through the pulmonary artery.
**FIG. 4B** is an image that shows a representative coronal image obtained by PET of rats administered ¹⁸F Rhodamine 6-G mitochondria (6 x 10⁷) delivered to the lungs by vascular infusion through the pulmonary artery.
**FIG. 4C** is an image that shows a representative image of lungs obtained by PET of rats administered ¹⁸F Rhodamine 6-G mitochondria (6 x 10⁷) delivered to the lungs by vascular infusion through the pulmonary artery.
**FIG. 5A** is an image of immunohistochemical staining of mouse lung tissue following administration of exogenous mitochondria by a nebulizer.
**FIG. 5B** is an image of immunohistochemical staining of mouse lung tissue following administration of exogenous mitochondria by the pulmonary artery.
**FIG. 6** are images of murine lungs following 2 hours acute lung injury (ALI) ischemia of the left lung and 24 hours recovery. The red circle indicates lungs that are subject to the ALI procedure. Right lung is indicted as R, the left lung is indicated as L. The heart (H) is shown in the middle.
**FIG. 7A** is a bar graph that shows lung resistance in the mouse lungs (n=4) subjected to 2 hours left lung ischemia following 24 hours recovery. Vehicle or Mitochondria were delivered intravascularly through the left pulmonary artery (V) or by nebulizer (N).
**FIG. 7B** is a bar graph that shows elastance in mouse lungs subjected to 2 hours left lung ischemia following 24 hours recovery. Vehicle or Mitochondria were delivered intravascularly through the left pulmonary artery (V) or by nebulizer (N).
**FIG. 8A** is a bar graph that shows quantification of circulating free mtDNA determined by real-time PCR on whole blood samples in BALB/cJ mice receiving respiration buffer only (Sham) or mitochondria (3 x 10⁷ of mitochondria in respiration buffer).
**FIG. 8B** are images of hematoxylin and eosin stained lung tissue.
**FIG. 8C** are images of Masson's trichrome stained lung tissue.
**FIG. 8D** are images of transmission electron microscopy of the lung tissues from the inferior right lobe.
**FIG. 9A** is an image that shows quantification of mitochondrial uptake and bio-distribution. PET-microCT imaging of ¹⁸F-Rhodamine 6G labeled mitochondria (1 x 10⁹) delivered to the lungs of Wistar rats both via injection to the pulmonary trunk.
**FIG. 9B** is an image that shows quantification of mitochondrial uptake and bio-distribution. PET-microCT imaging of 18F-Rhodamine 6G labeled mitochondria (1 x 10⁹) delivered to the lungs of Wistar rats as an aerosol via nebulization.
**FIG. 10A** is an immunohistochemical image (100x) demonstrating exogenous mitochondria from human cardiac fibroblast, delivered via vascular delivery being taken up in lung tissue.
**FIG. 10B** is an immunohistochemical image (100x) demonstrating exogenous mitochondria from human cardiac fibroblast, delivered via nebulization being taken up in lung tissue.
**FIG. 11A** is a bar graph that shows results of lung function analysis of dynamic compliance for lungs receiving mitochondria via vascular delivery.
**FIG. 11B** is a bar graph that shows results of lung function analysis of resistance for lungs receiving mitochondria via vascular delivery.
**FIG. 11C** is a bar graph that shows results of lung function analysis of tissue damping for lungs receiving mitochondria via vascular delivery.
**FIG. 11D** is a bar graph that shows results of lung function analysis of inspiratory capacity for lungs receiving mitochondria via vascular delivery.
**FIG. 11E** is a bar graph that shows results of lung function analysis of peak inspiratory pressure for lungs receiving mitochondria via vascular delivery.
**FIG. 12A** show results of lung function analysis of dynamic compliance for lungs receiving mitochondria via nebulization.
**FIG. 12B** is a bar graph that shows results of lung function analysis of resistance for lungs receiving mitochondria via nebulization.
**FIG. 12C** is a bar graph that shows results of lung function analysis of tissue damping for lungs receiving mitochondria via nebulization.
**FIG. 12D** is a bar graph that shows results of lung function analysis of inspiratory capacity for lungs receiving mitochondria via nebulization.
**FIG. 12E** is a bar graph that shows results of lung function analysis of peak inspiratory pressure for lungs receiving mitochondria via nebulization.
**FIG. 13** shows results of lung function analysis, in particular representative pressurevolume loops.
**FIG. 14A** are images that show hematoxylin and eosin (H&E), myeloperoxidase staining (MPO), TUNEL, and transmission Electron Microscope (EM) images of lung tissue sections for lungs receiving vehicle via vascular delivery; scale bars: 500 µm.
**FIG. 14B** are images that show hematoxylin and eosin (H&E), myeloperoxidase staining (MPO), TUNEL, and transmission Electron Microscope (EM) images of lung tissue sections for lungs receiving mitochondria via vascular delivery; scale bars: 500 µm.
**FIG. 14C** are images that show hematoxylin and eosin (H&E), myeloperoxidase staining (MPO), TUNEL, and transmission Electron Microscope (EM) images of lung tissue sections for lungs receiving vehicle via nebulization; scale bars: 500 µm.
**FIG. 14D** are images that show hematoxylin and eosin (H&E), myeloperoxidase staining (MPO), TUNEL, and transmission Electron Microscope (EM) images of lung tissue sections for lungs receiving mitochondria via nebulization; scale bars: 500 µm.
**FIG. 14E** are images that show hematoxylin and eosin (H&E), myeloperoxidase staining (MPO), TUNEL, and transmission Electron Microscope (EM) images of lung tissue sections for the sham group; scale bars: 500 µm.

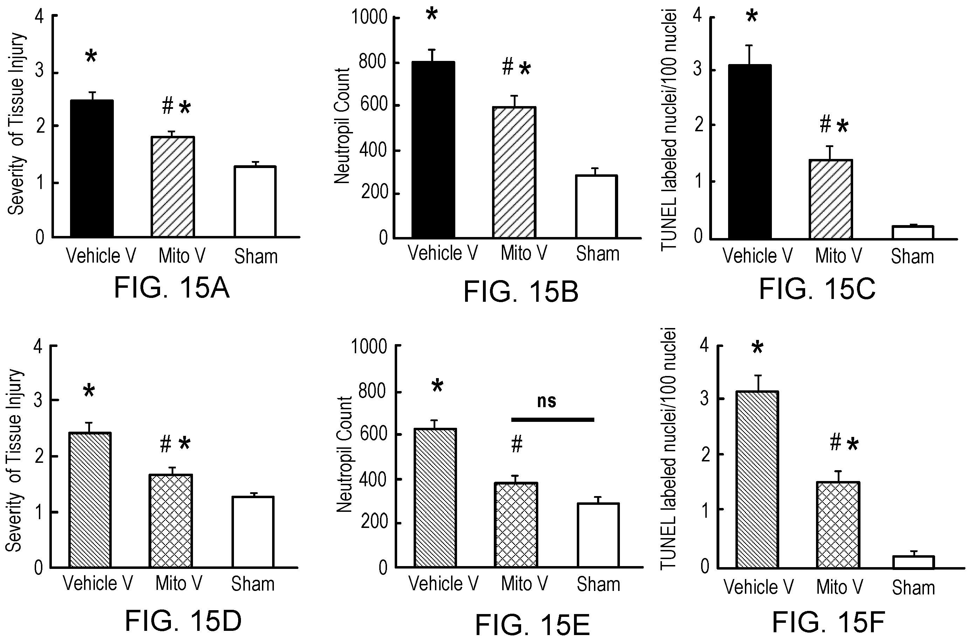

**FIG. 15A** is a bar graph showing severity of lung tissue injury at 24 hours of reperfusion for lungs receiving vehicle via vascular delivery (Vehicle V), mitochondria via vascular delivery (Mito V), and the sham group. Following 2 hours of ischemia and 24 hours of reperfusion, H&E stained sections (15x) were evaluated for severity of lung injury using previously described scoring system; the worse the tissue injury, the higher the score (1-5). Severity of tissue injury analysis show significantly decreased inflammatory cells infiltration and interstitial congestion with no signs of destruction of lung architecture in Mito V and Mito Neb lungs as compared to Vehicle V and Vehicle Neb.
**FIG. 15B** is a bar graph showing neutrophil count at 24 hours of reperfusion for lungs receiving vehicle via vascular delivery (Vehicle V), mitochondria via vascular delivery (Mito V), and the sham group. Following 2 hours of ischemia and 24 hours of reperfusion, H&E stained sections (15x) were evaluated for severity of lung injury using previously described scoring system; the worse the tissue injury, the higher the score (1-5). Severity of tissue injury analysis show significantly decreased inflammatory cells infiltration and interstitial congestion with no signs of destruction of lung architecture in Mito V and Mito Neb lungs as compared to Vehicle V and Vehicle Neb.
**FIG. 15C** is a bar graph showing TUNEL labeled nuclei per 100 nuclei at 24 hours of reperfusion for lungs receiving vehicle via vascular delivery (Vehicle V), mitochondria via vascular delivery (Mito V), and the sham group. Following 2 hours of ischemia and 24 hours of reperfusion, H&E stained sections (15x) were evaluated for severity of lung injury using previously described scoring system; the worse the tissue injury, the higher the score (1-5). Severity of tissue injury analysis show significantly decreased inflammatory cells infiltration and interstitial congestion with no signs of destruction of lung architecture in Mito V and Mito Neb lungs as compared to Vehicle V and Vehicle Neb.
**FIG. 15D** is a bar graph showing severity of lung tissue injury at 24 hours of reperfusion for lungs receiving vehicle via nebulization (Vehicle Neb), mitochondria via vascular delivery (Mito Neb), and the sham group. Following 2 hours of ischemia and 24 hours of reperfusion, H&E stained sections (15x) were evaluated for severity of lung injury using previously described scoring system; the worse the tissue injury, the higher the score (1-5). Severity of tissue injury analysis show significantly decreased inflammatory cells infiltration and interstitial congestion with no signs of destruction of lung architecture in Mito V and Mito Neb lungs as compared to Vehicle V and Vehicle Neb.
**FIG. 15E** is a bar graph showing neutrophil count at 24 hours of reperfusion for lungs receiving vehicle via nebulization (Vehicle Neb), mitochondria via vascular delivery (Mito Neb), and the sham group. Following 2 hours of ischemia and 24 hours of reperfusion, H&E stained sections (15x) were evaluated for severity of lung injury using previously described scoring system; the worse the tissue injury, the higher the score (1-5). Severity of tissue injury analysis show significantly decreased inflammatory cells infiltration and interstitial congestion with no signs of destruction of lung architecture in Mito V and Mito Neb lungs as compared to Vehicle V and Vehicle Neb.
**FIG. 15F** is a bar graph showing TUNEL labeled nuclei per 100 nuclei at 24 hours of reperfusion for lungs receiving vehicle via nebulization (Vehicle Neb), mitochondria via vascular delivery (Mito Neb), and the sham group. Following 2 hours of ischemia and 24 hours of reperfusion, H&E stained sections (15x) were evaluated for severity of lung injury using previously described scoring system; the worse the tissue injury, the higher the score (1-5). Severity of tissue injury analysis show significantly decreased inflammatory cells infiltration and interstitial congestion with no signs of destruction of lung architecture in Mito V and Mito Neb lungs as compared to Vehicle V and Vehicle Neb.

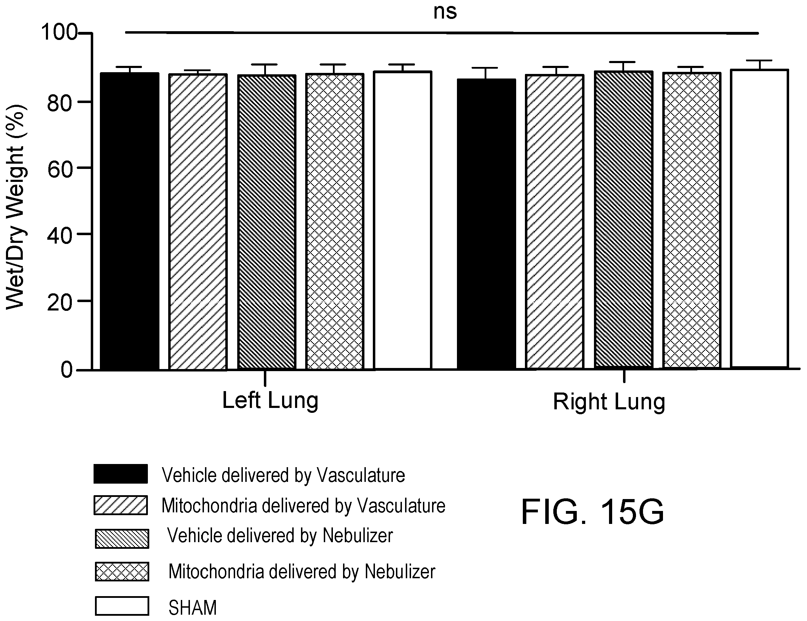

**FIG. 15G** is a bar graph showing wet to dry weight ratio in percent at 24 hours of reperfusion for lungs receiving vehicle via vascular delivery (Vehicle V), mitochondria via vascular delivery (Mito V), vehicle via nebulization (Vehicle Neb), mitochondria via vascular delivery (Mito Neb), and the sham group. Following 2 hours of ischemia and 24 hours of reperfusion, H&E stained sections (15x) were evaluated for severity of lung injury using previously described scoring system; the worse the tissue injury, the higher the score (1-5). Severity of tissue injury analysis show significantly decreased inflammatory cells infiltration and interstitial congestion with no signs of destruction of lung architecture in Mito V and Mito Neb lungs as compared to Vehicle V and Vehicle Neb.
**FIG. 16** show quantified results from BAL cytokine analysis; all values are expressed as mean ± SEM. n = 4 for all groups.

### DETAILED DESCRIPTION

This present disclosure relates to compositions containing mitochondria (e.g., viable and functional mitochondria), methods of preparing and using the compositions, and devices for administering the compositions to the respiratory tract of a subject. The present disclosure shows that mitochondria can be effectively administered to the subject through respiratory tract, and delivering the aerosolized form of the composition comprising mitochondria to respiratory tract is unexpectedly as effective as intra-artery administration.

The present disclosure also provides methods that can safely and reliably generate pharmaceutical compositions comprising viable and functional mitochondria. The pharmaceutical compositions as described herein can have increased therapeutic value, increased commercial value, and lower production cost per therapeutic dose.

In one aspect, the present disclosure provides methods of delivering mitochondria or combined mitochondrial agents to cells lining and surrounding the respiratory tract. Such pharmaceutical compositions can be taken up by cells, and once localized intracellularly, the mitochondria are able to confer one or more biological effects. These biological effects include, but are not limited to, producing the energy for the cell through respiration, producing ATP (i.e., converting ADP to ATP), regulating cellular metabolism, initiating citric acid cycle or the Krebs cycle, producing heat, storing calcium ions, signaling through mitochondrial reactive oxygen species, regulating the membrane potential, inducing apoptosis-programmed cell death, engaging in calcium signaling (e.g., calcium-evoked apoptosis), synthesizing heme and/or steroid, and initiating hormonal signaling, etc.

### Aerosolized compositions

The present disclosure provides aerosolized compositions comprising isolated mitochondria and/or combined mitochondrial agents. The present disclosure shows that cell organelles (e.g., mitochondria) in an aerosolized composition or in the form of aerosol can remain viable and functional, and thus can be effectively administered to the respiratory tract of a subject.

As used herein, the term "aerosol" refers to a suspension of fine particles or droplets in a carrier gas. As used herein, the term "aerosolization" refers to the process or the act of converting a composition into the form of an aerosol. The aerosolized composition comprises a plurality of particles (e.g., droplets, solid particles, liquid droplets, or hydrogel particles). In some embodiments, the particle has a size (e.g., a diameter) of about or less than 500 nm, 600 nm, 700 nm, 800 nm, 900 nm, 1 µm, 2 µm, 3 µm, 4 µm, 5 µm, 6 µm, 7 µm, 8 µm, 9 µm, 10 µm, 20 µm, 30 µm, 40 µm, 50 µm, 60 µm, 70 µm, 80 µm, 90 µm, 100 µm, 200 µm, 300 µm, 400 µm, 500 µm, 600 µm, 700 µm, 800 µm, 900 µm, or 1000 µm. In some embodiments, the particle has a size (e.g., a diameter) of about or greater than 500 nm, 600 nm, 700 nm, 800 nm, 900 nm, 1 µm, 2 µm, 3 µm, 4 µm, 5 µm, 6 µm, 7 µm, 8 µm, 9 µm, 10 µm, 20 µm, 30 µm, 40 µm, 50 µm, 60 µm, 70 µm, 80 µm, 90 µm, 100 µm, 200 µm, 300 µm, 400 µm, 500 µm, 600 µm, 700 µm, 800 µm, 900 µm, or 1000 µm. In some embodiments, the particle has a size (e.g., a diameter) of from 500 nm to 10 µm, from 500 nm to 100 µm, from 1 µm to 10 µm, from 1 µm to 100 µm, from 5 µm to 100 µm, from 10 µm to 100 µm, or from 1 µm to 500 µm. As used herein, the term "diameter" refers to the length of the longest straight line segment whose endpoints both lie on the surface of a particle.

In some embodiments, the aerosolized composition comprises particles (e.g., droplets) with different sizes. In some embodiments, about or at least 10%, 20%, 30%, 40%, 50%, 60%, 70%, 80%, 90%, 91%, 92%, 93%, 94%, 95%, 96%, 97%, 98%, or 99% of particles are from 500 nm to 10 µm, from 500 nm to 100 µm, from 1 µm to 10 µm, from 1 µm to 100 µm, from 5 µm to 100 µm, from 10 µm to 100 µm, or from 1 µm to 500 µm.

In some embodiments, the particles in the aerosolized composition can have a median size (e.g., a median diameter) of about or less than 500 nm, 600 nm, 700 nm, 800 nm, 900 nm, 1 µm, 2 µm, 3 µm, 4 µm, 5 µm, 6 µm, 7 µm, 8 µm, 9 µm, 10 µm, 20 µm, 30 µm, 40 µm, 50 µm, 60 µm, 70 µm, 80 µm, 90 µm, 100 µm, 200 µm, 300 µm, 400 µm, 500 µm, 600 µm, 700 µm, 800 µm, 900 µm, or 1000 µm. In some embodiments, the particles can have a median size (e.g., a median diameter) of about or greater than 500 nm, 600 nm, 700 nm, 800 nm, 900 nm, 1 µm, 2 µm, 3 µm, 4 µm, 5 µm, 6 µm, 7 µm, 8 µm, 9 µm, 10 µm, 20 µm, 30 µm, 40 µm, 50 µm, 60 µm, 70 µm, 80 µm, 90 µm, 100 µm, 200 µm, 300 µm, 400 µm, 500 µm, 600 µm, 700 µm, 800 µm, 900 µm, or 1000 µm. In some embodiments, the particles can have a median size (e.g., a median diameter) of from 500 nm to 10 µm, from 500 nm to 100 µm, from 1 µm to 10 µm, from 1 µm to 100 µm, from 5 µm to 100 µm, from 10 µm to 100 µm, or from 1 µm to 500 µm. As used herein, the term "median size" refers to the median size (e.g., diameter) as determined on a volume basis. Where the term median size is used, it is understood to describe the particle size that divides the population in half such that 50 % of the population on a volume basis is greater than or less than this size.

The aerosolized compositions can be made by various devices known in the art. Exemplary devices that can convert the composition into an aerosol form prior to administration include, e.g., a nebulizer, a vaporizer, a nasal sprayer, an inhaler, a soft mist inhaler, a jet nebulizer, an ultrasonic wave nebulizer, a pressurized metered dose inhaler, a breath activated pressurized metered dose inhaler, or a vibrating mesh device. These devices can use various carrier gases, including e.g., oxygen, air, nitrogen, or compressed air.

The composition before aerosolization can include one or more pharmaceutically acceptable carriers. A pharmaceutically acceptable carrier can include a compound or composition useful in facilitating storage, stability, administration, cell targeting and/or delivery of the mitochondria and/or combined mitochondrial agent. The pharmaceutically acceptable carrier include, without limitation, suitable vehicles, diluents, solvents, excipients, pH modifiers, osmolytes, salts, colorants, rheology modifiers, lubricants, coatings, fillers, antifoaming agents, polymers, hydrogels, surfactants, emulsifiers, adjuvants, preservatives, phospholipids, fatty acids, mono-, di- and tri-glycerides and derivatives thereof, waxes, oils and water. In some embodiments, isolated mitochondria and/or the combined mitochondrial agents are suspended in water, saline, buffer, respiration buffer, or sterile mitochondria buffer for delivery *in vivo.* Pharmaceutically acceptable salts, buffers or buffer systems, including, without limitation, saline, phosphate buffer, phosphate buffered saline (PBS) or respiration buffer can be included in a composition described herein. Vehicles having the ability to facilitate delivery to a cell *in vivo,* such as liposomes, may be utilized to facilitate delivery of mitochondria or the combined mitochondrial agents to the target cells.

Exemplary buffers include, but are not limited to, a respiration buffer (e.g., 250 mmol/L sucrose, 20 mmol/L K+-HEPES buffer, pH 7.2, 0.5 mmol/L K+-EGTA, pH 8.0). Exemplary osmolytes include, but are not limited to, trehalose, sucrose, mannose, glycine, proline, glycerol, mannitol, sorbitol, betaine, and sarcosine.

### Isolated mitochondria and combined mitochondrial agents

Mitochondria for use in the presently described methods can be isolated or provided from any source, e.g., isolated from cultured cells or tissues. Exemplary cells include, but are not limited to, muscle tissue cells, cardiac fibroblasts, cultured cells, HeLa cells, prostate cancer cells, yeast, blood cells, cultured cells, and among others, and any mixture thereof. Exemplary tissues include, but are not limited to, liver tissue, skeletal muscle, heart, brain, blood, and adipose tissue (e.g., brown adipose tissue). Mitochondria can be isolated from cells of an autogenous source, an allogeneic source, and/or a xenogeneic source. In some instances, mitochondria are isolated from cells with a genetic modification, e.g., cells with modified mtDNA or modified nuclear DNA.

Mitochondria can be isolated from cells or tissues by methods known to those of skill in the art. In some embodiments, tissue samples or cell samples are collected and then homogenized. Following homogenization, mitochondria are isolated by repetitive centrifugation. Alternatively, the cell homogenate can be filtered through nylon mesh filters. Typical methods of isolating mitochondria are described, for example, in McCully et al., Injection of isolated mitochondria during early reperfusion for cardioprotection, Am J Physiol 296, H94-H105. PMC2637784 (2009); Frezza et al., Organelle isolation: functional mitochondria from mouse liver, muscle and cultured filroblasts. Nature protocols, 2(2), 287-295 (2007); US20180057610; and US20180057610A1.

The mitochondria for the treatment can be isolated from cells or tissues of an autogenous source, an allogeneic source, and a xenogeneic source. In some instances, mitochondria are collected from cultured cells or tissues of a subject, and these mitochondria are administered back to the same subject. In some other cases, mitochondria are collected from cultured cells or tissues of a second subject, and these mitochondria are administered to a first subject. In some cases, mitochondria are collected from cultured cells or tissues from a different species (e.g., mice, swine, yeast).

The present disclosure also provides a composition comprising combined mitochondrial agent. The combined mitochondrial agents include e.g., mitochondria that are physically associated with an agent, such as a therapeutic agent, a diagnostic agent, and/or an imaging agent.

A therapeutic agent can be any agent that has a therapeutic or prophylactic use. Exemplary therapeutic agents include, e.g., therapeutic agents for ischemia-related disorders, cytotoxic agents for treating cancer, among many others. In some instances, mitochondria can deliver therapeutic agents to specific cells, for example, tumor cells. The therapeutic agent may be, e.g., an intracellular inhibitor, deactivator, toxin, arresting substance and/or cytostatic/cytotoxic substance that, once inside a cell, inhibits, destroys, arrests, modifies and/or alters the cell such that it can no longer function normally and/or survive. The therapeutic agent can be an agent to restore a cell's proper function, for example, a DNA vector for gene therapy. A therapeutic agent can be, e.g., an inorganic or organic compound; a small molecule (less than 500 daltons) or a large molecule; a proteinaceous molecule, such as a peptide, polypeptide, protein, post-translationally modified protein, or antibody; or a nucleic acid molecule, such as a double-stranded DNA, single-stranded DNA, double-stranded RNA, single-stranded RNA, or a triple helix nucleic acid molecule. In some embodiments, a therapeutic agent can be a natural product derived from any known organism (e.g., from an animal, plant, bacterium, fungus, protist, or virus) or from a library of synthetic molecules. In some embodiments, a therapeutic agent can be a monomeric or a polymeric compound. Some exemplary therapeutic agents include cytotoxic agents, DNA vectors, small interfering RNAs (siRNA), micro RNAs (miRNA), reactive peptides, nanoparticles, microspheres, and fluorescent molecules.

A diagnostic agent is an agent that has diagnostic use. As mitochondria carry a diagnostic agent into a cell, in some embodiments, the diagnostic agent can be designed to determine the condition within a cell, for example pH and oxidative stress within a cell.

An imaging agent is an agent that is employed for use in imaging techniques. The techniques or modalities include, but are not limited to, X-rays, computed tomography (CT), magnetic resonance imaging (MRI), scintigraphy, fluorescence, ultrasound, etc. The imaging agent can be florescent and/or radioactive. In some embodiments, an imaging agent can also be a diagnostic agent. Exemplary imaging agents include, but are not limited to, MitoTracker fluorophores (Thermo Fisher Scientific Inc.), CellLight^{®} RFP, BacMam 2.0 (Thermo Fisher Scientific Inc.), pH-sensitive pHrodo fluorescent dyes (Thermo Fisher Scientific Inc.), ¹⁸F-Rhodamine 6G, ¹⁸F-labeled rhodamine B, magnetic iron oxide nanoparticles, and gold- and platinum-based nanoparticles.

As discussed above, a combined mitochondrial agent comprises a mitochondria and an agent that are in direct and/or indirect physical contact with each other. For example, an agent can be linked to mitochondria, attached to mitochondria, embedded in the mitochondrial membrane, or completely or partially enclosed in mitochondria. In some instances, a pharmaceutical agent can be linked to mitochondria covalently. In some instances, the agent is linked to constituents of mitochondrial membrane directly through a covalent bond (e.g., a carboxamide bond and a disulfide bond), or indirectly through a linker (e.g., a peptide linker) or another covalently bonded agent. In other instances, an agent can be linked to mitochondria non-covalently, for example, through hydrophobic interaction, Van der Waals interaction, and/or electrostatic interaction, etc.

In some embodiments, a combined mitochondrial agent can comprise two or more different types of agents, for example, two different kinds of therapeutic agents, three different kinds of imaging agents, one therapeutic agent and one imaging agent, a therapeutic agent and a diagnostic agent, etc. Skilled practitioner will appreciate that any variation is possible.

One particularly useful linker to link mitochondria and an agent provides a sustained release of the agent upon injection. This can be accomplished, for example, using a hydrazone functional group. For example, a hydrazone is formed to covalently bind an agent to constituents on the mitochondrial membrane. Once this combined mitochondrial agent is taken up by cells, the change in pH will result in hydrolysis of the hydrazone, releasing the bound agent inside the cell.

In some embodiments, a therapeutic agent, a diagnostic agent, and/or an imaging agent can be linked to the outer mitochondrial membrane using functionalized surface chemistry. In some cases, heterobifunctional chemistries can link a therapeutic agent, a diagnostic agent, and/or an imaging agent to the mitochondrial surface, and once they are internalized, these agents can be released through interactions with intercellular esterases (e.g. via interaction with an acetoxymethyl ester) or through a UV-light activation or Near-Infrared light activation strategy. The UV-light activation and Near-Infrared light activation strategies are described, e.g., in Zhou et al., "Progress in the Field of Constructing Near-Infrared Light-Responsive Drug Delivery Platforms," Journal of Nanoscience and Nanotechnology 16.3 (2016): 2111-2125; Bansal et al., "Photocontrolled nanoparticle delivery systems for biomedical applications," Accounts of chemical research 47.10 (2014): 3052-3060; Barhoumi et al., "Ultraviolet light-mediated drug delivery: Principles, applications, and challenges," Journal of Controlled Release 219 (2015): 31-42; and US20180057610A1.

Skilled practitioners will appreciate that in some instances a composition described herein may include more than one type of combined mitochondrial agent. For example, included are compositions comprising mitochondria wherein essentially each mitochondrion is associated with multiple types of agents. Also included are compositions comprising mitochondria wherein each mitochondrion is paired with only one type of agent but wherein the composition comprises a mixture of mitochondria/agent pairings.

### Methods of making combined mitochondrial agents

Skilled practitioners will appreciate that an agent can be linked to mitochondria in any number of ways, e.g., by attaching to mitochondria, embedding partially or completely in the mitochondrial membrane, enclosing in mitochondria, or encapsulating within the mitochondria.

While not intending to be bound by any theory or any particular approach, it is believed that the outer membrane of mitochondria is adherent and thus particularly amenable to combination with various agents. In some embodiments, pharmaceutical agents can be attached to the outer membrane of mitochondria simply by incubation. For example, an effective amount of pharmaceutic agents can be fully mixed with isolated mitochondria in a buffer, e.g., respiration buffer, at a temperature favorable to isolated mitochondria, e.g., from 0 °C to 26 °C, from 0 °C to 4 °C, or about 0 °C, 4°C, 26 °C. This procedure is useful to attach an effective amount of pharmaceutic agents (e.g., nanoparticles, DNA vectors, RNA vectors) to mitochondria.

In some embodiments, organic cations (e.g., rhodamine and tetramethylrosamine) are readily sequestered by functioning mitochondria because of the electric potential on mitochondrial membrane. Healthy mitochondrial membranes maintain a difference in electric potential between the interior and exterior of the organelle, referred to as the membrane potential. This membrane potential is a direct result of mitochondrial functional processes, and can be lost if the mitochondria are not working properly. Lipid-soluble cations are sequestered by mitochondria as a consequence of their positive charge and of their solubility in both the inner membrane lipids and the matrix aqueous space. Similarly, in some other embodiments, anions can be attached to the outer membrane of mitochondria because of its negative charge. To link mitochondria with these pharmaceutical agents, an effective amount of pharmaceutic agents should be fully mixed with isolated mitochondria in a buffer, e.g., respiration buffer, at a temperature favorable to isolated mitochondria, e.g., about 0°C or 4°C.

The therapeutic, diagnostic, and/or imaging agent can be linked to phospholipids, peptides, or proteins on the mitochondrial membrane through a chemical bond. For example, molecules including fluorophores (pHrodo Red (Thermo Fisher Scientific, Inc.)) and metallic particles (e.g., 30 nm magnetic iron oxide nanoparticles (Sigma)) can be covalently linked to exposed amine groups on proteins and peptides exposed on the outside membrane of intact mitochondria using succinimidyl ester conjugates. These reactive reagents react with nonprotonated aliphatic amine groups, including the amine terminus of proteins and the ε-amino group of lysine residues, which creates a stable carboxamide bond. In another example, when the pharmaceutic agent, e.g., MitoTracker^{®} Orange CMTMRos (Invitrogen, Carlsbad, CA, now Thermo-Fisher Scientific, Cambridge, MA), are mixed with functional mitochondria, they are oxidized and then react with thiols on proteins and peptides on mitochondria to form conjugates.

There are numerous reactive chemical moieties available for attaching therapeutic, diagnostic, and/or imaging agents to the surface of mitochondria (e.g. carboxylic acid, amine functionalized, etc.).

Agents can be attached via protein bonding, amine bonding or other attachment methods either to the outer or inner mitochondrial membrane. Alternatively, or in addition, an agent can be attached to the mitochondria membrane through hydrophobic interaction, Van der Waals interaction, and/or electrostatic interaction.

In many instances, therapeutic agents, diagnostic agents and imaging agents may simply be mixed with isolated mitochondria, and incubated in a buffer (e.g., respiration buffer) for a sufficient period of time (e.g., a few minutes, 5 minutes, 10 minutes, or 1 hour) at favorable conditions (e.g., from 0 °C to 26 °C, from 0 °C to 4 °C, or about 0 °C, 4°C, 26 °C, pH 7.2~8.0).

Exemplary methods of preparing combined mitochondrial agents are described in McCully et al, Injection of isolated mitochondria during early reperfusion for cardioprotection, Am J Physiol 296, H94-H105. PMC2637784 (2009); and Masuzawa et al, Transplantation of autologously derived mitochondria protects the heart from ischemia-reperfusion injury, Am J Physiol 304, H966-982. PMC3625892 (2013); and US20180057610A1.

### Methods of treatment

The methods described herein include methods for the treatment of various diseases (e.g., lung disorders, respiratory disorders), ischemia reperfusion injury, and various other diseases described herein. Generally, the methods include administering a therapeutically effective amount of a composition comprising isolated mitochondria or combined mitochondria agent as described herein, to a subject who is in need of, or who has been determined to be in need of, such treatment. In some embodiments, the composition is administered through respiratory tract.

As used in this context, to "treat" means to ameliorate at least one symptom of the disorder (e.g., shortness of breath, fast breathing, low oxygen level in the blood, lung failure, respiratory failure, heart failure (e.g., right heart failure)). In some embodiments, the treatment results in improvement in pulmonary functions (e.g., increase of oxygen level in the blood, reducing resistance, reducing elastance). In some embodiments, the methods described herein can be used to treat pulmonary fibrosis, lung infection (e.g., by coronavirus), asthma, chronic obstructive pulmonary disease, pulmonary emboli, lung cancer, pulmonary hypertension, or lymphangioleiomyomatosis. In some embodiments, the methods described herein can be used to treat pneumonia.

The terms "subject" and "patient" are used interchangeably throughout the specification and describe an animal, human or non-human, to whom treatment according to the methods of the present invention is provided. Veterinary and non-veterinary applications are contemplated by the present invention.

A human patient can be an adult or a child, such as a newborn child. For example, the human subject can be at least or about 60 years old, e.g., at least about 65 years old, 70 years old, 75 years old, or at least or about 80 years old. The human subject can be below or about the age of 18 years old, e.g., 15 years old, 10 years old, 9 years old, 8 years old, 7 years old, 6 years old, 5 years old, 4 years old, 3 years old, 2 years old, or below or about 1 year old.

In addition to humans, subjects can include, but are not limited, non-humans, e.g., mice, rats, hamsters, guinea-pigs, rabbits, ferrets, cats, dogs, and primates. Included are, for example, non-human primates (e.g., monkey, chimpanzee, gorilla, and the like), rodents (e.g., rats, mice, gerbils, hamsters, ferrets, rabbits), lagomorphs, swine (e.g., pig, miniature pig), equine, canine, feline, bovine, and other domestic, farm, and zoo animals.

Administering the compositions as described herein can be accomplished by various means, for example, intravenous, intradermal, subcutaneous, transdermal (topical), transmucosal, rectal administration. nasal administration, nasal instillation, insufflation (e.g., nasal sprays), inhalation (through nose or mouth), intrapulmonary administration, intratracheal administration, intraperitoneal injection, infusion, or any combinations thereof. In some embodiments, the composition is administered through respiratory tract.

In one aspect, the isolated mitochondria and/or combined mitochondrial agents can be delivered to lungs or heart to decrease stunning and allow for weaning of the organs from a surgical procedure (e.g., lung surgery or heart surgery). In some embodiments, the methods involve administering isolated mitochondria and/or combined mitochondrial agents through the respiratory tract.

In one aspect, the isolated mitochondria and/or combined mitochondrial agents can be delivered to ears, eyes, skin (e.g., for reducing wrinkles), or head (e.g., for baldness).

The compositions described herein can be administered to a subject as a singular, onetime treatment, or alternatively, multiple treatments, e.g., a treatment course that continues intermittently or continuously for about 1, 2, 5, 8, 10, 20, 30, 50, or 60 days, one year, indefinitely, or until a health care provider determines that administration of the mitochondria or combined mitochondrial agent is no longer necessary.

Isolated mitochondria and/or combined mitochondrial agents can be administered to a subject every as a single dose or multiple dosed delivered every 5 min or at 12-24 hours. In some embodiments, isolated mitochondria or combined mitochondrial agents can be administered to a subject every 5-10 minutes (e.g., every 5 minutes, every 10 minutes).

It is noted that in some cases, the effects of mitochondria depend on the length of the time period between the time of isolation and the time of use. Thus, in some instances, the mitochondria are freshly isolated and viable. The mitochondria or combined mitochondrial agents can be administered to a subject within about 5 minutes, about 10 minutes, about 20 minutes, about 30 minutes, about 40 minutes, about 50 minutes, about 60 minutes, about 70 minutes, about 80 minutes, about 90 minutes, about 100 minutes, about 110 minutes, about 120 minutes after the mitochondria are isolated. In some instances, the mitochondria or combined mitochondrial agents are administered to a subject within about 5 minutes, about 10 minutes, about 20 minutes, about 30 minutes, about 40 minutes, about 50 minutes, about 60 minutes, about 70 minutes, about 80 minutes, about 90 minutes, about 100 minutes, about 110 minutes, about 120 minutes after starting the mitochondria isolating process. Mitochondria and/or combined mitochondrial agents may in some instances be stored for a short period of time (e.g., about or at least 10 minutes, 20 minutes, 30 minutes, 40 minutes, 50 minutes, or 60 minutes) before use.

It is also noted that, in some cases, frozen-thawed mitochondria are not viable and not effective for certain treatments described herein, e.g., treatment of ischemia/reperfusion injuries. Thus, in some cases, the mitochondria are not frozen and thawed after isolation from tissues and/or cells.

In some embodiments, the compositions and the methods described herein can be used to treat disorders associated with lungs, ears, eyes, skin, scalp. For example, the compositions and the methods described herein can be used to treat cancers, wrinkle (e.g., wrinkles on skins), or baldness. In some cases, the compositions and the methods described herein to enhance the functions of various tissues and organs (e.g., lungs, ears, eyes, skin).

### Administration through respiratory tract

The mucus (1-10 µm thick) that lines the pulmonary epithelium and the surfactant that lines the alveoli (0.1-0.2 µm thick) constitute physical barriers to pulmonary absorption of foreign particles. Furthermore, membrane-associated (epithelial and endothelial) and intracellular (macrophages, lymphocytes, neutrophils and mast cells) proteases and peptidases readily degrade administered peptides and proteins (Agu et al., "The lung as a route for systemic delivery of therapeutic proteins and peptides." Respiratory research 2.4 (2001): 198). The pulmonary macrophages have also been shown to secrete or release shortlived peroxidases, inflammatory and immunomodulatory mediators (including granulocyte colony-stimulating factor, interleukins, leukotrienes and proteases), and other molecules as part of a host defense mechanism.

Nevertheless, the present disclosure shows that administering a composition comprising mitochondria or combined mitochondria agents through the respiratory tract can effectively promote lung function, reduce lung resistance, reduce lung elastance, reduce ischemic area, and/or reduce edema and cellular damage in the lungs.

The compositions as described herein can be administered through respiratory tract by various means, for example, nasal administration, nasal instillation, insufflation (e.g., nasal sprays), inhalation (through nose or mouth), intrapulmonary administration, intratracheal administration, or any combinations thereof. As used herein, the term "nasal instillation" refers to a procedure that delivers a therapeutic agent directly into the nose and onto the nasal membranes, wherein a portion of the therapeutic agent can pass through tracheas and is delivered into the lung.

Because of the occasionally limited functionally for the lungs that are in need of treatment, a therapeutic agent sometimes cannot be effectively delivered to the target sites in the lungs (e.g., bronchioles or alveoli) through respiratory tract administration. In these cases, an agent that can clear the airways can be administered to the subject first. In some embodiments, these agents can induce dilation of bronchial passages, and/or vasodilation in muscle. Such agents include, but are not limited to, beta2 adrenergic receptor agonists, anticholinergic agents, corticosteroids. In some embodiments, an agent for treating asthma can be used.

Pharmaceutical compositions suitable for administering through respiratory tract can include, e.g., liquid solutions, aqueous solutions (where water soluble), or dispersions, etc. In some embodiments, these compositions can comprise one or more surfactants.

As used herein, the term "respiratory tract" refers to the air passages from the nose to the pulmonary alveoli, including the nose, throat, pharynx, larynx, trachea, bronchi, and any part of the lungs. In some embodiments, the composition is administered to the lungs or any part of the respiratory system.

In some embodiments, the compositions can be administered a subject by a delivery system that can convert the composition into an aerosol form, e.g., a nebulizer, a vaporizer, a nasal sprayer, an inhaler, a soft mist inhaler, a jet nebulizer, an ultrasonic wave nebulizer, a pressurized metered dose inhaler, a breath activated pressurized metered dose inhaler, or a vibrating mesh device. As used herein, the term "inhaler" refers to a device for administering compositions in the form of a spray or dry powder that is inhaled (breathed in either naturally or mechanically forced in to the lungs) through the nose or mouth. In some embodiments, inhalers include e.g., a passive or active ventilator (mechanical with or without an endotracheal tube), nebulizer, dry powder inhaler, metered dose inhaler, and pressurized metered dose inhaler. Once mitochondria are deposited or localized near cells, a subset of the mitochondria can be taken up by the cells.

In some embodiments, the devices can use air (e.g., oxygen, compressed air) or ultrasonic power to break up solutions and suspensions into small aerosol particles (e.g., droplets) that can be directly inhaled from the mouthpiece of the device. In some embodiments, the devices use a mesh/membrane with laser drilled holes (e.g., from 1000 to 7000 holes) that vibrates at the top of the liquid reservoir, and thereby pressures out a mist of very fine droplets through the holes.

The delivery system can also have a unit dose delivery system. The volume of solution or suspension delivered per dose can be anywhere from about 5 to about 2000 microliters, from about 10 to about 1000 microliters, or from about 50 to about 500 microliters. Delivery systems for these various dosage forms can be dropper bottles, plastic squeeze units, atomizers, nebulizers or pharmaceutical aerosols in either unit dose or multiple dose packages.

In some embodiments, the device is a small, hard bottle to which a metered dose sprayer is attached. The metered dose can be delivered by drawing the composition into a chamber of defined volume, which chamber has an aperture dimensioned to aerosolize and aerosol formulation by forming a spray when a liquid in the chamber is compressed. The chamber is compressed to administer the composition. In certain devices, the chamber is a piston arrangement. Such devices are commercially available.

Alternatively, a squeeze bottle with an aperture or opening dimensioned to aerosolize an aerosol formulation by forming a spray when squeezed can be used. The opening is usually found in the top of the bottle, and the top is generally tapered to partially fit in the nasal passages for efficient administration of the aerosol formulation. Preferably, the nasal inhaler can provide a metered amount of the aerosol formulation, for administration of a measured dose of the therapeutic agent.

In some embodiments, the aerosolization of a liquid formulation for inhalation into the lung involves a propellant. The propellant may be any propellant generally used in the art. Specific non-limiting examples of such useful propellants are a chlorofluorocarbon, a hydrofluorocarbon, a hydrochlorofluorocarbon, or a hydrocarbon, including trifluoromethane, dichlorodifluoromethane, dichlorotetrafluoroethanol, and 1,1,1,2-tetrafluoroethane, or combinations thereof.

The present disclosure also provides aerosol formulations and dosage forms for use in treating subjects with various lung diseases. In general, such dosage forms contain the therapeutic agent in a pharmaceutically acceptable diluent. Pharmaceutically acceptable diluents in such aerosol formulations include but are not limited to sterile water, saline, buffered saline, dextrose solution, and the like. In certain embodiments, a diluent that may be used in the present invention or the pharmaceutical formulation is phosphate buffered saline or a buffered saline solution generally between the pH 7.0-8.0 range (e.g., pH 7.4), or water.

The aerosol formulation also may optionally include pharmaceutically acceptable carriers, diluents, solubilizing or emulsifying agents, surfactants and excipients.

The formulation can include a carrier. The carrier is a macromolecule which is soluble in the circulatory system and which is physiologically acceptable where physiological acceptance means that those of skill in the art would accept injection of said carrier into a patient as part of a therapeutic regime. The carrier preferably is relatively stable in the circulatory system with an acceptable plasma half-life for clearance. Such macromolecules include but are not limited to soy lecithin, oleic acid and sorbitan trioleate.

The formulations can also include other agents useful for pH maintenance, solution stabilization, or for the regulation of osmotic pressure. Examples of the agents include but are not limited to salts, such as sodium chloride, or potassium chloride, and carbohydrates, such as glucose, galactose or mannose, and the like.

The present disclosure further contemplates aerosol formulations comprising the composition as described herein and another therapeutically effective agent.

In some embodiments, the composition can be administered into blood vessels. The blood can carry the mitochondria or the combined mitochondria agent to a target site, for example, an organ, a tissue, or an injured site. For example, pulmonary blood vessels (e.g., pulmonary veins) can carry the mitochondria or the combined mitochondrial agent to the lung. In some cases, the composition described herein can be used to improve the function of the lung, ears, eyes, and skins.

In some embodiments, the compositions are administered by the oral or nasal respiratory route for local or systemic effect. Compositions in pharmaceutically acceptable solvents can be nebulized by use of inert gases. Nebulized solutions can be inhaled directly from the nebulizing device or the nebulizing device can be attached to a face mask, or intermittent positive pressure breathing machine. Solution, suspension, or powder compositions can be administered, orally or nasally, from devices that deliver the formulation in an appropriate manner. For administration by inhalation, the compositions can be delivered in the form of an aerosol spray from a pressured container or dispenser that contains a suitable propellant, e.g., a gas such as carbon dioxide, or a nebulizer. Such methods include those described in U.S. Patent No. 6,468,798; U.S. Patent No. 6,695,227; U.S. Patent No. 7,431,222; US20050224608.

Thus, in some embodiments, the aerosol composition can be administered to respiratory track, lungs, ears, eyes, nasal passage, rectum, skins, and head. In some embodiments, the composition is delivered to lungs, nasal passage, rectum, intestine, skin, and eyes to treat cancers or tumors in these sites.

In some embodiments, the composition is administered through a metered dose sprayer, a squeeze bottle, a pressurized metered dose inhaler, a ventilator, or face mask and tent. Systems of aerosol delivery, such as the pressurized metered dose inhaler and the dry powder inhaler are disclosed in Newman, S. P., Aerosols and the Lung, Clarke, S. W. and Davia, D. editors, pp. 197-22. The subject can also inhale the composition through a ventilator, which will be set to a flow rate based on patient comfort and needs. This is determined by pulmonary graphics (i.e., respiratory rate, tidal volumes, etc.). The composition can also be prepared to allow inhalation by the subject using a facemask or tent. In certain cases, the composition can be packaged into a portable device (e.g., a portable inhaler device) and administered in a metered dose, for example, to permit intermittent treatment of a recipient who is not in a hospital setting. Different concentrations of composition can be packaged in the containers.

A convenient way to effectively deliver the composition to the alveoli is to select a nebulizer which produces sufficiently small particles (e.g., producing particles with a mean particle diameter of less than 5.0 microns (µm), more preferably having a mean particle diameter of about 0.2 to about 4.0 µm, and most preferably having a mean diameter of about 0.2 to about 2 µm). As an alternative to selecting small mean particle diameters to achieve substantial alveoli deposition, a very high dosage of the composition can be administered, with a larger mean particle diameter. In such an approach, it is beneficial if the particular composition is not too irritating at the required dosage and that there are a sufficient number of particles in the total population having a diameter in the 0.5 to about 5 µm range to allow for deposition in the alveoli. For proximal airway delivery, the mean particle size may be larger. For example, suitable mean particle diameters may generally be less than about 15 µm, e.g., from about 4 µm to about 15 µm, e.g., from about 5 µm to about 10 µm. The composition can be aerosolized by any appropriate method. For use with humans or other primates, the aerosol can be generated using a medical nebulizer system which delivers the aerosol through a mouthpiece, facemask, etc., from which the subject can draw the aerosol into the lungs. Various nebulizers are known in the art and can be used in the method as described herein. See, e.g., U.S. Pat. No. 4,268,460; U.S. Pat. No. 4,253,468; U.S. Pat. No. 4,046,146; U.S. Pat. No. 3,826,255; U.S. Pat. No. 4,649,911; U.S. Pat. No. 4,510,829. The selection of a nebulizer system will depend on whether alveolar or airway delivery (i.e., trachea, primary, secondary or tertiary bronchi, etc.) is desired.

Examples of nebulizers useful for alveolar delivery include the Acorn 1 nebulizer, and the Respirgard II^{™} Nebulizer System, both available commercially from Marquest Medical Products, Inc., Inglewood, Colo. Other commercially available nebulizers for use with the instant invention include the UltraVent^{™} nebulizer available from Mallinckrodt, Inc. (Maryland Heights, Mo.); the Wright nebulizer (Wright, B. M., Lancet (1958) 3:24-25); and the DeVilbiss nebulizer (Mercer et al., Am. Ind. HYR. Assoc. J. (1968) 29:66-78; T. T. Mercer, Chest (1981) 80:6 (Sup) 813-817). Nebulizers useful for airway delivery include those typically used in the treatment of asthma. Such nebulizers are also commercially available. One of skill in the art can determine the usefulness of a particular nebulizer by measuring the mean particle size generated thereby with e.g. a 7 stage Mercer cascade impactor (Intox Products, Albuquerque, N. Mex.).

The total amount of the composition delivered to the subject will depend upon several factors, including the total amount aerosolized, the type of nebulizer, the particle size, subject breathing patterns, severity of lung disease, and concentration in the aerosolized solution, and length of inhalation therapy. The amount of composition measured in the alveoli may be substantially less than what would be expected to be from the amount of composition present in the aerosol, since a large portion of the composition may be exhaled by the subject or trapped on the interior surfaces of the nebulizer apparatus.

Skilled practitioners will be able to readily design effective protocols, particularly if the particle size of the aerosol is optimized. In some instances, it is useful to administer higher doses when treating more severe conditions. If necessary, the treatment can be repeated on an *ad hoc* basis depending upon the results achieved. If the treatment is repeated, the mammalian host can be monitored to ensure that there is no adverse immune response to the treatment. The frequency of treatments depends upon a number of factors, such as the amount of composition administered per dose, as well as the health and history of the subject. As used herein, the "amount nebulized" or "amount aerosolized" of the composition means the amount that actually leaves the apparatus as an aerosol, i.e., the amount placed into the apparatus less the amount retained in the reservoir and on the inner surfaces of the apparatus at the conclusion of a treatment session.

### Lung Disorders

The therapeutic agents described herein can be used to treat various lung disorders. The subject can have any lung disease that may benefit from the treatment, e.g., ischemia, perfusion injury, pulmonary hypoplasia, congenital diaphragmatic hernia (CDH), Poland syndrome, Chest wall diseases (e.g., pectus excavatum), pneumonectomy, bronchopulmonary dysplasia, lung injury, and inhalation injury, asthma, cystic fibrosis, etc. For example, the subject may have pulmonary hypoplasia. In some embodiments, an effective amount of composition comprising isolated mitochondria can be administered to the subject, for example, through respiratory tract.

In some embodiments, the methods described herein can be used to treat acute respiratory distress syndrome (ARDS), acute lung injury (ALI), respiratory failure, reduced respiratory function, and/or lung inflammation.

In some embodiments, the methods described herein can be used to treat pulmonary fibrosis, lung infection (e.g., by virus, coronavirus, flu, bacteria, or fungi), asthma, chronic obstructive pulmonary disease, pulmonary emboli, lung cancer, pulmonary hypertension, or lymphangioleiomyomatosis. In some embodiments, the methods described herein can be used to treat pneumonia.

In some embodiments, the subject may be identified as having insufficient, incomplete, or slow lung development. Alternatively or in addition, the subject may have some damage in the lungs. For example, the subject can be a habitual smoker or a worker frequently exposed to smoke inhalation.

### Treating ischemia-related injuries

Methods described herein can be used to treat ischemic lungs. For example, an effective amount of isolated mitochondria can be administered through the respiratory tract. The mitochondria can be internalized by the cells in the lungs.

Reperfusion injury is the tissue damage by blood supply when blood returns to the tissue after a period of ischemia or lack of oxygen. The absence of oxygen and nutrients during the ischemic period results in inflammation and oxidative damage when blood flow is restored. The inflammatory response further leads to the reperfusion injury in the tissue. Therefore, in some instances, a treatment also involves administering immune suppressors to the patient. The immune suppressors can be e.g., administrated separately, but as a concurrent treatment with the mitochondrial agent. Alternatively, or in addition, the immune suppressors can be linked to mitochondria to form a combined mitochondrial agent, which can be used for the treatment. Particularly useful immune suppressors are bisphosphonates.

In some embodiments, the subject has acute lung injury (ALI). Acute lung injury is often associated with acute respiratory failure and refractory hypoxemia. In some embodiments, the subject has bilateral pulmonary infiltrates that are consistent with pulmonary edema in the absence of left atrial hypertension. ALI complicates a wide variety of medical and surgical conditions and reflects the pulmonary response to lung insult or precipitating factors. The most common reason for ALI is ischemial reperfusion injury (IRI). IRI can occur with extracorporeal gas exchange, cardiopulmonary bypass and lung ventilation and has been shown to have profound effects on lung function and cellular viability and to significantly contribute to increased morbidity and mortality in both pediatric and adult patients. The incidence of ALI in adult patients undergoing cardiac surgery has been estimated to range from 53.8% to 73.5%. In pediatric patients, ALI is present in 8-10% of patients requiring mechanical ventilation. In both pediatric and adult patients, ALI has an estimated mortality of 20-75%. At present, there are no viable methodologies to treat ALI occurring through IRI with a goal to increase lung survival and lung function.

Methods described herein can be used to treat ALI. For example, an effective amount of aerosolized composition comprising mitochondria or combined mitochondrial agents can be administered to the respiratory tract of a subject. For example, about or at least 1 × 10⁷ of mitochondria can be administered into the lung. The injected mitochondria are internalized by endothelial cells and can provide enhanced oxygen consumption, reduce lung resistance and elastance, increase compliance, and/or reduce ischemic area.

### Vasodilation and blood flow

The methods described herein can significantly increase blood flow without altering mean blood pressure or heart rate. The ability to increase blood flow with no increase in heart rate allows for clinical usage in angina type injury and in ischemia/reperfusion related injury and in tissue damage areas where increased blood flow and oxygen delivery would be needed. Thus, the methods described herein can be used in artery interventions to remove clots or obstructions in blood vessels.

Methods described herein can also be used to increase blood flow and/or oxygen delivery for various organs or tissues (e.g., heart, or lungs), and induce vascular dilatation in the organs. In some instances, the isolated mitochondria or combined mitochondrial agents can be used to decrease vascular resistance in an organ (e.g., heart, or lung). Isolated mitochondria or combined mitochondrial agents can be used to increased blood flow. The isolated mitochondria and/or combined mitochondrial agents can be added to a contrast agent, and can be used in the identification and removal of blockages in the organs.

It is noted that the effects of the compositions are dependent on time from isolation to time of use. The vasodilatory effects decreases as time from isolation is extended. While not intending to be bound by any theory, it is hypothesized that freshly isolated mitochondria have certain chemicals, which can increase blood flow. Therefore, in some methods, the mitochondria are freshly isolated and viable. For example, the mitochondria or combined mitochondrial agents are administered to a subject within about 5 minutes, about 10 minutes, about 20 minutes, about 30 minutes, about 40 minutes, about 50 minutes, about 60 minutes, about 70 minutes, about 80 minutes, about 90 minutes, about 100 minutes, about 110 minutes, about 120 minutes after the time point when the mitochondria isolation process starts or after the mitochondria are isolated. In some cases, the mitochondria or combined mitochondrial agents are administered to a subject within about 20 minutes to about 60 minutes (e.g., about 20 minutes, about 30 minutes, about 40 minutes, about 50 minutes, about 60 minutes) after the time point when the mitochondria isolation process starts or after the mitochondria are isolated.

In some cases, increasing blood flow is not desirable (e.g., treating ischemia/reperfusion in lungs, treating cancer). In these cases, mitochondria or combined mitochondrial agents can be stored for a short period of time (e.g., from about 30 to about 60 minutes) before usage. This method can be used to increase tissue viability (e.g., treating ischemia/reperfusion injury) without causing an increase in blood flow. In these cases, the mitochondria or combined mitochondrial agents are administered to a subject at least 60 about minutes (e.g., about 65 minutes, about 70 minutes, about 80 minutes, about 90 minutes, about 100 minutes, about 110 minutes, about 120 minutes) after the time point when the mitochondria isolation process starts or after the mitochondria are isolated.

### Imaging

Imaging agents can be attached to mitochondria, often by co-incubation of the mitochondria with the imaging agents. Such imaging agents include, but are not limited to, MitoTracker and pHrodo fluorophores from Thermo Fisher Scientific Inc., ¹⁸F-Rhodamine 6G, and iron oxide nanoparticles.

Combined mitochondrial agents that include an imaging agent can be administered to the subject through respiratory tract. Tissues containing the labeled mitochondria can be examined using imaging techniques, such as positron emission tomopgrahy (PET), microcomputed tomography (µCT), and magnetic resonance imaging (MRI), brightfield microscope, and 3-D super-resolution microscopy, etc. Skilled practitioners will appreciate that other imaging techniques or modalities may be used. They include, but are not limited to, x-rays, scintigraphy, fluorescence and ultrasound.

Positron emission tomography is an imaging technique that produces a three-dimensional image in the body, and can be used in methods described herein. The system detects pairs of gamma rays emitted indirectly by a positron-emitting radioisotope (tracer). Three-dimensional images of tracer concentration within the body are then constructed by computer analysis. Useful reporter groups include radioactive isotopes, such as ¹¹C, ¹³N, ¹⁵O, ¹⁸F, ⁶⁴Cu, ⁶⁸Ga, ⁸¹mKr, ⁸²Rb, ⁸⁶Y, ⁸⁹Zr, ¹¹¹In, ¹²³I, ¹²⁴I, ¹³³Xe, ²⁰¹Tl, ¹²⁵I, ³⁵S ¹⁴C, ³H. In some methods, mitochondria can be labeled by radioactive isotopes, e.g., ¹⁸F, or by molecules that incorporate radioactive isotopes, e.g., ¹⁸F-Rhodamine 6G, ¹⁸F-labeled rhodamine B. After the mitochondria are internalized by target cells, the PET imaging technique, or similar technique, can be employed to view the target cells.

Magnetic resonance imaging is a medical imaging technique to image the anatomy and the physiological processes of the body and can be used in methods described herein. In some instances, it can be used in conjugation with some other imaging techniques, for example, PET. Images acquired from both devices can be taken sequentially, in the same session, and combined into a single superposed (co-registered) image. PET/MRI scans can be used to diagnose a health condition in humans and animals, e.g., for research, medical, and agricultural purposes.

Micro-computed tomography uses x-rays to create cross-sections of a physical object that can be used to recreate a virtual model without destroying the original object, and can be used in methods described herein. In some instances, it is used in conjugation with some other imaging techniques, for example, PET. Images acquired from both devices can be taken sequentially, in the same session, and combined into a single superposed (co-registered) image. Thus, functional imaging obtained by PET, which depicts the spatial distribution of metabolic or biochemical activity in the body can be more precisely aligned or correlated with anatomic imaging obtained by CT scanning. Two- and three-dimensional image reconstruction may be rendered as a function of a common software and control system.

3D-structured illumination microscopy, 3D-SIM, or 3-D super-resolution microscopy, allows complete 3D visualization of structures inside cells and can be used in the methods described herein. Structured illumination microscopy is an imaging method capable of doubling the spatial resolution of conventional widefield fluorescence microscopy by using spatially structured illumination light. It enhances spatial resolution by collecting information from frequency space outside the observable region.

The described methods, i.e., methods that include administering mitochondria and/or combined mitochondrial agents, are useful for diagnosing a variety of diseases, such as cancers, (e.g., lung, brain, pancreatic, melanoma, prostate, colon cancers), cardiovascular disease (e.g., myocardial infarction, atherosclerosis), autoimmune diseases (e.g., multiple sclerosis, diabetes, irritable bowel syndrome, Celiac disease, Crohn's disease), and inflammatory disease.

Methods using agents for imaging purpose are well-known in the art and described in, for example, Bartholomä et al., Biological characterization of F18-labeled Rhodamine B, a potential positron emission tomography perfusion tracer, Nucl Med Biol 40, 1043-1048, PMC3820364 (2013); Bartholomä et al., 18F-labeled rhodamines as potential myocardial perfusion agents: comparison of pharmacokinetic properties of several rhodamines, Nucl Med Biol 42, 796-803, PMC4567415 (2015): and Pacak et al., Superparamagnetic iron oxide nanoparticles function as a long-term, multi-modal imaging label for non-invasive tracking of implanted progenitor cells, PLoS ONE 9, e108695, PMC4177390 (2014).

### Drug delivery

The present specification provides methods to deliver pharmaceutic agents, e.g., to cells and/or tissues of a patient. Mitochondria are taken up by tissue cells through an actin-dependent internalization process, thereby providing a way to deliver pharmaceutic agents directly into the cells. In some instances, combined mitochondrial agents enter into lung tissues or blood vessels through the respiratory tract.

An antibody or an antigen-binding fragment can be linked or attached to mitochondria. Skilled practitioners will appreciate that linking the antibody or antigen binding fragment to mitochondria or combined mitochondrial agent can allow the mitochondria or combined mitochondrial agent to be targeted to specific sites, e.g., to target cells and/or tissues. In some instances, the antibody or the antigen-binding fragment are designed to target specific cell types, for example, smooth muscle cells in lung, immune cells, macrophages, etc.

In some embodiments, the present disclosure provides methods of delivering a nucleic acid (e.g., DNA, RNA, miRNA), a vector, a peptide, or a protein to a target site (e.g., lung tissue). Isolated mitochondria can be used as a carrier to deliver a nucleic acid or a peptide into a cell. In some instances, combined mitochondrial agents that include nucleic acid polymers can be administered to a subject to replace a mutated gene in the subject that causes disease, to inactivate, or "knock out," a mutated gene, or to introduce a new gene into the subject. Exemplary nucleic acid polymers include, but are not limited to, double-stranded DNA, single-stranded DNA, double-stranded RNA, single-stranded RNA, or triple helix nucleic acid molecules. In certain instances, the nucleic acid polymers are DNA, interfering RNAs (siRNA), and micro RNAs.

### Minimizing cardiotoxicity

Chemotherapy is a common treatment for various cancers, however, it also causes several serious complications. Chemotherapy-induced cardiotoxicity is one complication that limits the clinical use of chemotherapeutic agents. Certain chemotherapeutic agents, such as anthracyclines, are highly effective against acute lymphoblastic and myeloblastic leukemias, but are particularly harmful to the heart due to its effects on mitochondria. The damage to mitochondria further leads to chemotherapy-induced cardiotoxicity. Angsutararux et al., Chemotherapy-Induced Cardiotoxicity: Overview of the Roles of Oxidative Stress. Oxid Med Cell Longev. 2015;2015:795602. doi: 10.1155/2015/795602 (2015); Guo et al., Cardiovascular toxicities from systemic breast cancer therapy, Front Oncol. 4:346. doi: 10.3389/fonc.2014.00346. eCollection (2014).

The present disclosure provides methods to minimize chemotherapy-induced cardiotoxicity. The methods involve administering an effective amount of isolated mitochondria and/or a combined mitochondrial agent to a patient through respiratory tract. If the patient needs to be treated with chemotherapy (e.g., because prescribed by a physician or veterinarian), the patient can be treated with mitochondria and/or combined mitochondrial agent, before, during, and/or after administration of the chemotherapy. For example, patients can be treated with mitochondria and/or combined mitochondrial agent starting immediately after administration, as a singular treatment or continuing intermittently or continuously for about 1, 2, 5, 8, 10, 20, 30, 50, or 60 days, one year, indefinitely, or until a physician determines that administration of the mitochondria and/or combined mitochondrial agent is no longer necessary.

### Organ/tissue transplantation

The present disclosure also features methods of transplanting an organ(s), tissues, masses of cells and/or isolated cells. The methods can include a step of exposing the organ(s), tissues, mass of cells and/or isolated cells to mitochondria or combined mitochondrial agents prior to transplantation. Such exposures can occur *in situ* and/or *ex vivo.* The organ(s), tissues and/or isolated cells may be exposed to a composition comprising mitochondria or combined mitochondrial agents (e.g., an aerosolized composition).

Exposure of an organ or tissue to compositions comprising mitochondria or combined mitochondrial agents can be performed *ex vivo* and/or *in situ* by any method known in the art. For example, the exposure may be performed *ex vivo* in any chamber or space having sufficient volume for submerging the organ or tissue, completely or partially, in the composition. As another example, the organ may be exposed to compositions comprising mitochondria or combined mitochondrial agents by placing the organ in any suitable container, and causing the compositions comprising mitochondria or combined mitochondrial agents to "wash over" the organ, such that the organ is exposed to a continuous flow of the composition.

An effective amount of mitochondria or combined mitochondria agents is an amount that is effective for enhancing survival and/or improving function of organs, or cells *in vivo* and/or *in vitro.* Within the context of transplantation of individual cells or masses of cells, e.g., transplant donors and/or recipients, an effective amount of mitochondria or combined mitochondria agents is an amount that is administered to the transplant donor and/or recipient sufficient to enhance survival of the cell or mass of cells, e.g. to reduce loss of the cell, or mass of cells, and/or to improve functional performance of a transplanted cell or a mass of cells. Within the context of transplantation of organs and tissues, e.g., transplant donors and/or recipients, an effective amount of mitochondria or combined mitochondria agents is an amount that is administered to the transplant donor and/or recipient sufficient to enhance survival of the organ, tissue or cells of interest, e.g., to reduce loss of cells from which the organ or tissue is composed, and/or to improve functional performance of an organ.

In some instances, the administration of the composition through a respiratory tract is performed before the organ is retrieved from the donor. In some instances, the administration of the composition through a respiratory tract is performed after the organ is transplanted into the recipient. In some instances, administration of the composition (e.g., aerosolized composition) are performed before organ retrieval, after harvesting of the organ, and then again after implantation into the recipient. In some instances, the administration of the composition is performed during the transplantation surgery (e.g., lung transplantation, heart transplantation).

### Treatment of cancer

Methods described herein also provide treatment of cancers (e.g., lung cancers). After the compositions as described herein are administered to the subject through respiratory tract, the mitochondria or the combined mitochondrial agents can be taken up by the tumor cells. In some embodiments, a cytostatic agent or cytotoxic agent can be delivered to the tumor to kill cancer cells. In some embodiments, the therapeutic agent is a chemotherapeutic agent, for example, anthracycline.

Cancers that may be treated using the methods and compositions of the present disclosure include, e.g., mouth/pharynx cancer, esophagus cancer, larynx cancer, lung cancer, or any cancer in the respiratory tract.

Further, in some embodiments, an antibody or an antigen-binding fragment can be linked or attached to mitochondria. Skilled practitioners will appreciate that linking the antibody or antigen binding fragment to mitochondria or combined mitochondrial agents can allow the mitochondria or combined mitochondrial agents to target specific sites, e.g., to target cells and/or tissues. In some instances, the antibody or the antigen-binding fragment are designed to target specific cell types, for example, cancer cells.

### Dosage

An "effective amount" is an amount sufficient to effect beneficial or desired results. For example, a therapeutic amount is one that achieves the desired therapeutic effect. This amount can be the same or different from a prophylactically effective amount, which is an amount necessary to prevent onset of disease or disease symptoms. An effective amount can be administered in one or more administrations, applications or dosages. A therapeutically effective amount of a therapeutic agent (i.e., an effective dosage) depends on the therapeutic agents selected. The compositions can be administered one from one or more times per day to one or more times per week; including once every other day. The skilled artisan will appreciate that certain factors may influence the dosage and timing required to effectively treat a subject, including but not limited to the severity of the disease or disorder, previous treatments, the general health and/or age of the subject, and other diseases present. Moreover, treatment of a subject with a therapeutically effective amount of the therapeutic agents described herein can include a single treatment or a series of treatments.

Dosage, toxicity and therapeutic efficacy of the therapeutic agents can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Agents which exhibit high therapeutic indices are preferred. While agents that exhibit toxic side effects may be used, care should be taken to design a delivery system that targets such agents to the site of affected tissue in order to minimize potential damage to uninfected cells and, thereby, reduce side effects. How to estimate a safe range for a human patient is described, e.g., in Food and Drug Administration. "Guidance for industry: estimating the Attorney Docket No.:36443-0037EP2 maximum safe starting dose in initial clinical trials for therapeutics in adult healthy volunteers." Center for Drug Evaluation and Research (CDER) (2005).

The data obtained from cell culture assays and animal studies can be used in formulating a range of dosage for use in humans. The dosage of such agents lies preferably within a range of circulating concentrations that include the ED50 with little or no toxicity. The dosage may vary within this range depending upon the dosage form employed and the route of administration utilized. For any agent used in the method of the invention, the therapeutically effective dose can be estimated initially from cell culture assays. Such information can be used to more accurately determine useful doses in humans. Levels in plasma may be measured, for example, by high performance liquid chromatography.

Skilled practitioners will appreciate that the amount of mitochondria and/or combined mitochondrial agents, e.g., compositions comprising mitochondria and/or combined mitochondrial agents, that should be administered to a patient will vary depending upon, e.g., the type of disorder being treated, the route of administration, the duration of the treatment, the size of an area to be treated, and/or the location of the treatment site in the patent, among others. Skilled practitioners will be able to determine dosages to be administered depending on these and other variables. For example, a total of about 1 x 10 ¹⁰ to 1 x 10 ¹⁴ of mitochondria can be administered to a subject (e.g., to treat localized ischemia in the lungs). In the case of small focal lesions, 1 x 10 ³ to 1 x 10 ⁶ mitochondria can be administered to the patient. Therefore, an effective amount of mitochondria or combined mitochondrial agents (or compositions comprising same) is the total amount of mitochondria or combined mitochondrial agents sufficient to bring about a desired therapeutic effect. An effective amount can be, e.g., at least or about 1 x 10² mitochondria or combined mitochondrial agents e.g., from about 1 x 10³ to about 1 x 10¹⁴, about 1 x 10⁴ to about 1 x 10¹³, about 1 x 10⁵ to about 1 x 10¹², about 1 x 10⁶ to about 1 x 10¹¹, about 1 x 10⁷ to about 1 x 10¹⁰, about 1 x 10³ to about 1 x 10⁷, about 1 x 10⁴ to about 1 x 10⁶, about 1 x 10⁷ to about 1 x 10¹⁴, or about 1 x 10⁸ to about 1 x 10¹³, about 1 x 10⁹ to about 1 x 10¹², about 1 x 10⁵ to about 1 x 10⁸ ; or at least or about 1 x 10³, 1 x 10⁴, 1 x 10⁵, 1 × 10⁶, 1 x 10 ⁷, 1 x 10 ⁸, 1 x 10 ⁹, 1 x 10 ¹⁰, 1 x 10 ¹¹, 1 x 10¹², 1 x 10¹³, or at least or about 1 x 10¹⁴, or e.g., an amount more than 1 x 10 ¹⁴. As used herein, the term "total amount" in the context of administration to a patient can refer to the total amount of mitochondria or combined mitochondrial agents in a single administration or in multiple administrations, depending on the dosing regimen being performed.

### Pharmaceutical compositions

The disclosure provides compositions that comprise isolated mitochondria, compositions that comprise combined mitochondrial agents, compositions that comprise both isolated mitochondria and combined mitochondrial agents, and methods of using such compositions.

A pharmaceutical composition described herein may include mitochondria and/or combined mitochondria agents and a pharmaceutically acceptable carrier. As used herein, the language "pharmaceutically acceptable carrier" includes saline, solvents, dispersion media, coatings, antibacterial and antifungal agents, isotonic and absorption delaying agents, and the like, compatible with pharmaceutical administration. In some embodiments, the pharmaceutically acceptable carrier is phosphate buffered saline, saline, Krebs buffer, Tyrode's solution, contrast media, or omnipaque, or a mixture thereof. In some embodiments, the pharmaceutically acceptable carrier is sterile mitochondria buffer (300 mM sucrose; 10 mM K+-HEPES (potassium buffered (4-(2-hydroxyethyl)-1-piperazineethanesulfonic acid, pH 7.2); 1 mM K+-EGTA, (potassium buffered ethylene glycol tetraacetic acid, pH 8.0)). In some embodiments, the pharmaceutically acceptable carrier is respiration buffer (250 mM sucrose, 2 mM KH₂PO₄, 10 mM MgCl₂, 20 mM K-HEPES Buffer (pH 7.2), and 0.5 mM K-EGTA (pH 8.0)).

Pharmaceutical compositions are typically formulated to be compatible with its intended route of administration.

A pharmaceutical composition can be formulated for various clinical uses, e.g., imaging, treating wounds, treating injuries, preserving organs, improving mitochondrial functions in organs or tissues, and skin care. In some cases, the pharmaceutically acceptable carrier is a contrast agent for imaging purpose. In some embodiments, the pharmaceutical composition may include antiseptic agents, antibacterial agents (e.g., antibiotics), antifungal agents, disinfectants, analgesic agents, anesthetic agents, steroids, nutritional supplements, ethereal oils, etc. An anesthetic agent is a drug that can prevent pain during surgery or treatment. Exemplary analgesic agents include, without limitation, paracetamol, nonsteroid anti-inflammatory drugs, salicylates, ibuprofen and lidocaine. Exemplary antibacterial agents include, without limitation, dichlorobenzyl alcohol, amylmetacresol and antibiotics. Exemplary antibiotics include penicillins carbapenems, cephalosporins aminoglycosides, bacitracin, gramicidin, mupirocin, chloramphenicol, thiamphenicol, lincomycin, clindamycin, macrolides, novobiocin, polymyxins, rifamycins, spectinomycin, tetracyclines, vancomycin, teicoplanin, streptogramins, anti- folate agents, sulfonamides, trimethoprim, pyrimethamine, nitrofurans, methenamine mandelate, methenamine hippurate, nitroimidazoles, quinolones, fluoroquinolones, isoniazid, ethambutol, pyrazinamide, para-aminosalicylic acid, cycloserine, capreomycin, ethionamide, prothionamide, thiacetazone and viomycin. Antiseptic agents are antimicrobial substances that can be applied to living tissue/skin to reduce the possibility of infection, sepsis, or putrefaction. Exemplary antiseptics include, without limitation, chlorhexidine and salts thereof, benzalkonium and salts thereof, triclosan and cetylpyridium chloride. Exemplary antifungal agents include, without limitation, tolnaftate, miconazole, fluconazole, clotrimazole, econazole, ketoconazole, itraconazole, terbinafine, amphotericin, nystatin and natamycin. Exemplary steroids include, without limitation, prednisone acetate, prednisone valerate, prednisolone, alclometasone dipropionate, fluocinolone acetonide, dexamethasone, methylprednisolone, desonide, pivolate, clocortolone pivolate, triamcinolone acetonide, prednicarbate, fluticasone propionate, flurandrenolide, mometasone furoate, desoximetasone, betamethasone, betamethasone dipropionate, betamethasone valerate, betamethasone propionate, betamethasone benzoate, diflorasone diacetate, fluocinonide, halcinonide, amcinonide, halobetasol propionate, and clobetasol propionate. Exemplary nutritional supplements include, without limitation, vitamins, minerals, herbal products and amino acids. Vitamins include without limitation, vitamin A, those in the vitamin B family, vitamin C, those in the vitamin D family, vitamin E and vitamin K. Ethereal oils include without limitation, those derived from mint, sage, fir, lavender, basil, lemon, juniper, rosemary, eucalyptus, marigold, chamomile, orange and the like. Many of these agents are described, e.g., in WO 2008152626.

Compositions comprising mitochondria and/or combined mitochondrial agents can be formulated in any form, e.g., liquids, semi-solids, or solids. Exemplary compositions include liquids, creams, ointments, salves, oils, emulsions, liposome formulations, among others.

Isolated mitochondria or combined mitochondrial agents can be included in compositions that are designed for use in organ, tissue, or cell transplantation. The composition may include isolated mitochondria and/or combined mitochondrial agents and a liquid that is suitable for administration to patients and/or organs *in situ* or *ex vivo,* e.g., for maintaining organs, tissues or cells *ex vivo.* In general, the liquid will be an aqueous solution. Examples of solutions include Phosphate Buffered Saline (PBS), Celsior^{™} solution, Perfadex^{™} solution, Collins solution, citrate solution, tissue culture media (e.g., Dulbecco's Modified Eagle's Medium (DMEM)), the Histidine-tryptophan-ketoglutarate (HTK) solution, and the University of Wisconsin (UW) solution (Oxford Textbook of Surgery, Morris and Malt, Eds., Oxford University Press, 1994).

The University of Wisconsin cold storage solution is considered a standard solution for organ transplantation. It includes the following: 100 mM potassium lactobionate, 25 mM KH₂PO₄, 5 mM MgSO₄, 30 mM raffinose, 5 mM adenosine, 3 mM glutathione, 1 mM allopurinol, and 50 g/L hydroxyethyl starch. Isolated mitochondria or combined mitochondrial agents can be added to these liquids for organ, tissue and cell preservation.

When administered through certain routes, such as the respiratory tract (e.g., nose, a tracheostomy, or by endotracheal tube (ETT)), surfactants can allow the composition to reach the distal airways. Thus, in some embodiments, compositions described herein (e.g., liquid solutions, aqueous solutions (where water-soluble), aerosol formulations, dispersions, sterile powders etc.) can comprise one or more surfactants.

The surfactant can be selected from the group consisting of non-ionic, cationic, anionic, and zwitterionic surfactants. Mixtures of surfactants can also be used. Exemplary classes of surfactants include alcohol ether sulfates, alcohol sulfates, alkanolamides, alkyl sulfonates, amine oxides, amphoteric surfactants, anionic surfactants, betaine derivatives, cationic surfactants, disulfonates, dodecylbenzene, sulfonic acid, ethoxylated alcohols, ethoxylated alkyl phenols, ethoxylated fatty acids, glycerol esters hydrotropes, lauryl sulfates, mono and diglycerides, non-ionic surfactants, phosphate esters, quaternary surfactants, and sorbitan derivatives.

To be suitable for delivery through respiratory tract, some other appropriate pharmaceutical carriers can also be used. These carries include e.g., liposomes, nano- and microparticles, cyclodextrins, microemulsions, micelles, suspensions, or solutions. In some embodiments, these carriers are made of lipids (e.g., liposomes, niosomes, microemulsions, lipidic micelles, solid lipid nanoparticles) or composed of polymers (e.g., polymer micelles, dendrimers, polymeric nanoparticles, nonogels, nanocapsules). These carriers can carry the therapeutic agent to the lung in a controlled manner. Nanocarriers can also be used. Localized therapy of the target organ generally requires smaller total doses to achieve clinically effective results. To reach this goal, nanocarriers can be engineered to slowly degrade, react to stimuli and to be site specific.

Pharmaceutical compositions described herein can be included in a container, pack, or dispenser together with instructions for administration. Pharmaceutical compositions described herein can also be prepared in a form that is suitable to be converted to an aerosolized form (e.g., by an appropriate nebulizer).

### EXAMPLES

The invention is further described in the following examples, which do not limit the scope of the invention described in the claims.

### Example 1: Isolating mitochondria from autologous skeletal muscle from subject

Mitochondria were isolated from autologous skeletal muscle tissue. The source of tissue was dependent upon the surgical entry point and access. Highly efficacious mitochondrial transplantation typically requires freshly isolated, viable, and functional mitochondria. Non-viable mitochondria (e.g., previously frozen mitochondria), mitochondrial fractions (proteins, complex I-V), mitochondrial DNA and RNA, and exogenous ATP or ADP usually do not have sufficient functionality.

### Methods

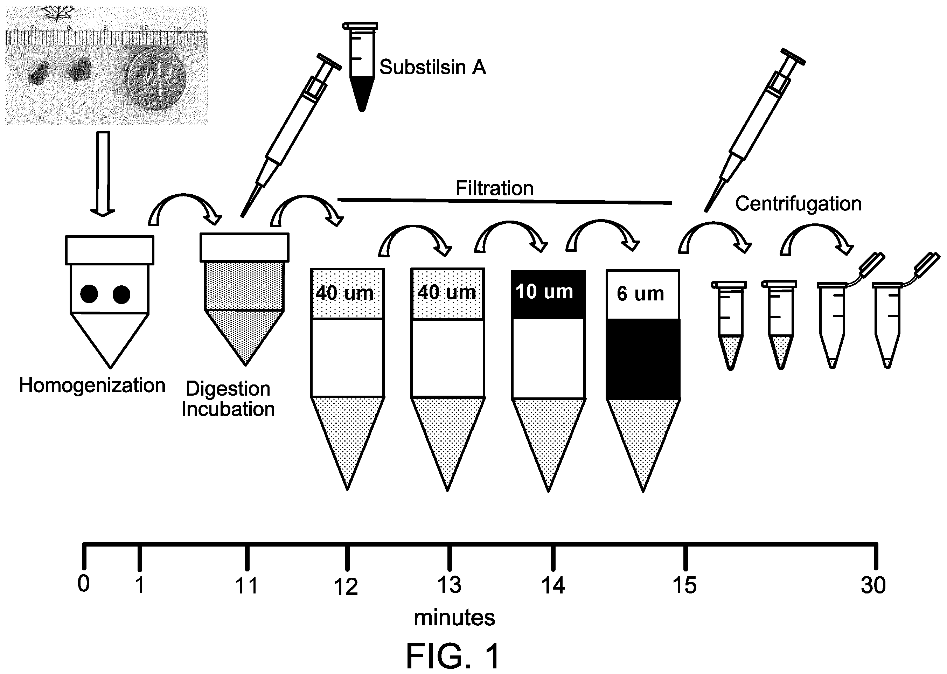

To allow for the isolation of viable respiration competent mitochondria, a rapid method was developed for the isolation and purification of mitochondria that can be performed in 20-30 minutes (FIG. 1). The major advantages of this method are: (1) it requires only a small amount of tissue that can be isolated using a #6 biopsy punch (<0.01 g tissue); (2) it uses a standardized tissue dissociation process that allows for uniform and consistent homogenization of tissue that is not easily achieved with manual homogenization methods; and (3) the use of differential filtration, rather than centrifugation, eliminates time-consuming and repetitive centrifugation steps and shortens the preparation time. In brief, two small pieces of skeletal muscle tissue (< 0.1 gram) were obtained using a #6 biopsy punch. The tissue was homogenized in a volume of 5 mL of isolation buffer (300 mmol/L sucrose, 10 mmol/L HEPES-KOH, 1 mmol/L EGTA-KOH, pH 7.4) and then incubated for 10 minutes with Subtilisin A enzymatic on ice. The digested tissue was then filtered through a series of filters pre-wetted with isolation buffer, and the mitochondria were subsequently precipitated by centrifugation at 9 x G for 5 minutes at 4 °C.

### Results

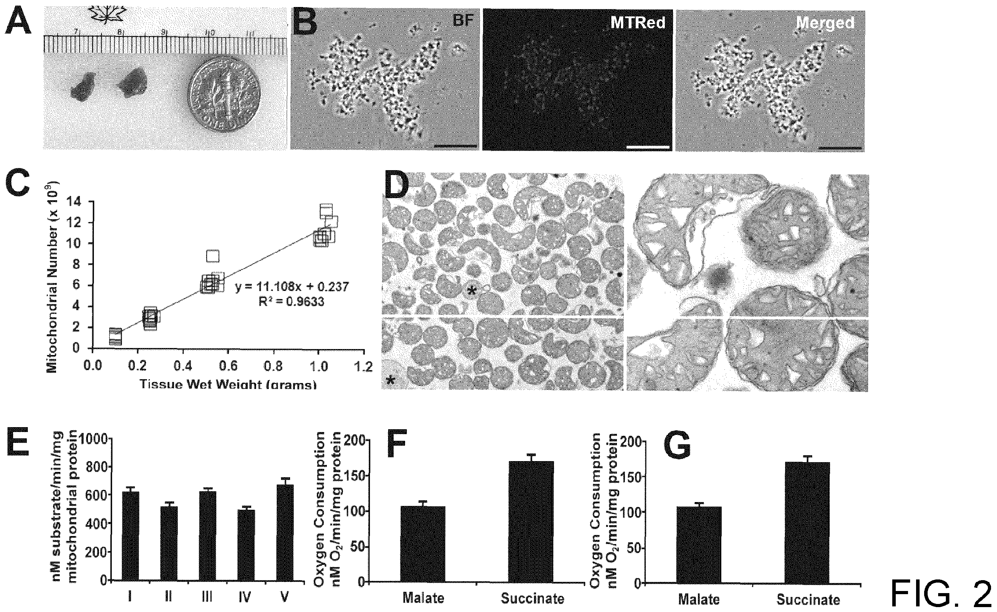

FIGS. 2A-2G shows representative results for isolation of mitochondria from skeletal muscle. FIG. 2A shows skeletal muscle tissue that was obtained using the biopsy punch. FIG. 2B shows representative microscopic images of isolated mitochondria under phase contrast illumination (bright field, BF) (left panel) and under fluorescence labeled with MitoTracker Red CMXRos (middle panel). The merged image is shown on the right panel. The mitochondria obtained by this method on average have greater than 99% viability. FIG. 2C shows representative results for mitochondrial yield per gram tissue wet weight. FIG. 2D shows representative transmission electron microscopy images of isolated mitochondria. Isolated mitochondria were electron dense with less than 0.01% of the mitochondria being fractured or damaged (*). FIG. 2E shows representative activity results for the mitochondrial complex I-V. FIG. 2F shows the representative results for the state 3 (active) oxygen consumption (ADP-stimulated respiration). FIG. 2G shows representative results for the respiratory control index (RCI; state 3/state 4) for malate induced (complex I) and succinate induced (complex II) oxygen consumption in energized autologous pectoralis major mitochondria. The data indicated that the isolated mitochondria maintain membrane potential and viability. Additionally, the data indicated that oxygen consumption and respiratory control index for malate-induced complex I and succinate-induced complex II were equal to that of mitochondria isolated by some other methods.

### Example 2: Isolated mitochondria were present and viable for at least 28 days following injection into the myocardium

Once isolated by the method as described in Example 1, the mitochondria were immediately used for mitochondrial transplantation.

### Mitochondrial Dosage

Experiments were performed to determine the optimal dose of autologous mitochondria. These experiments have demonstrated that 2 x 10⁵ mitochondria per gram of tissue wet weight provides optimal efficacy.

### Delivery of Isolated Mitochondria

The isolated mitochondria were delivered either by direct injection into the ischemic region of the lung by vascular infusion or by a nebulizer.
(a). Direct Injection of mitochondria provides localized distribution of mitochondria. The mitochondria were injected into the pulmonary artery or the right atrium using a tuberculin syringe with a 28g needle.
(b). Intravascular delivery of mitochondria simplifies mitochondrial delivery to tissue and allows for widespread distribution of autologous mitochondria within the tissue.
(c). Nebulizer delivery of mitochondria. To increase applicability and allow for early, non-surgical intervention and post-surgical intervention, the autologous mitochondria were delivered by a nebulizer to the lungs.

Vascular delivery provides localized delivery. Preliminary experiments were performed to demonstrate the safety and efficacy of the delivery of autologous mitochondria by intravascular delivery through the right atrium and through the pulmonary artery. The results of the preliminary experiments demonstrated that autologous mitochondrial transplantation delivered through either of these routes provides tissue specific delivery and uptake of mitochondria and is safe and efficacious, significantly enhancing pulmonary function following 2 hours ischemia and 24 hours recovery.

### Results

Fluorescence microscopy and MRI have demonstrated that transplanted mitochondria were present and viable for at least 28 days following injection into the myocardium. The majority of the mitochondria were found initially within the interstitial spaces between cells. Within 30 minutes post-delivery, the transplanted mitochondria were internalized within the cells by endocytosis and were seen within cardiomyocytes residing near the sarcolemma between Z-lines of the sarcomeres and in clusters around endogenous damaged mitochondria as well as near the nucleus. Enumeration of injected mitochondria has shown that 43.52% ± 4.46 (mean ± SEM) of the injected mitochondria are attached to or found within cardiomyocytes. Three-dimensional super-resolution microscopy and transmission electron microscopy were used to determine the intracellular fate of endocytosed exogenous mitochondria in human iPS-derived cardiomyocytes and primary cardiac fibroblasts. The results showed that isolated mitochondria were incorporated into cardiac cells within minutes and then transported by endocytosis into the cells where they fused with the endogenous mitochondrial network.

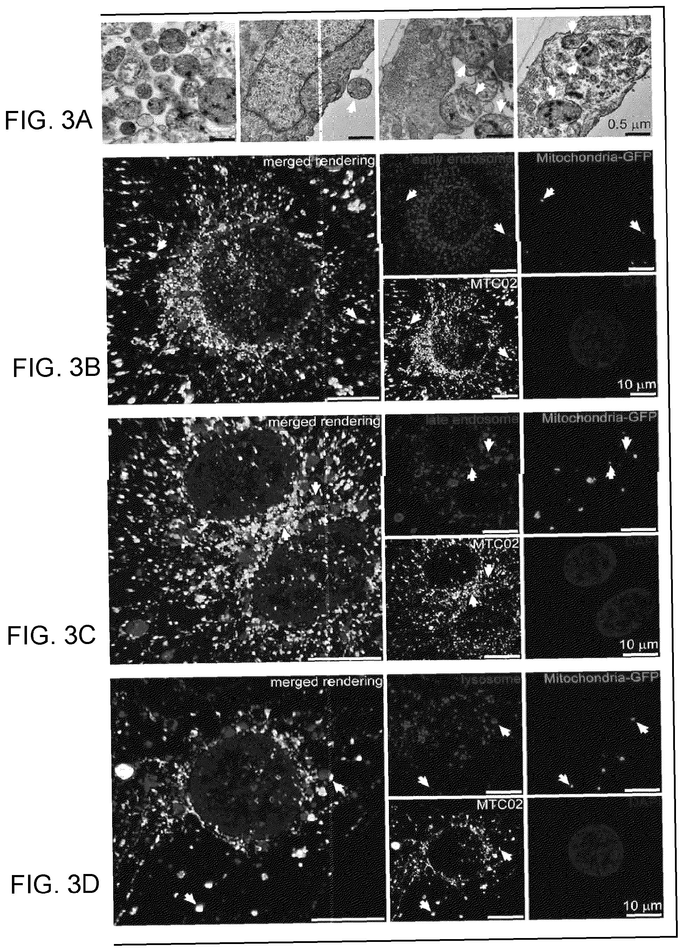

FIGS. 3A-3D show representative results of uptake and endocytic transport of extracellular mitochondria in cardiomyocytes. FIG. 3A shows representative images of iPS-CMs that were exposed to gold-labelled HCF mitochondria for 2.5, 5, and 10 min and imaged by TEM. The labelled mitochondria had electron dense deposits and were apparent at all three study times outside cells, adjacent to the apical cell surface, undergoing endocytosis, and inside cells (left to right and indicated by arrows). Images are representative of four experiments at 5 min; scale bars, 0.5 µm. FIG. 3B shows representative images from four color 3-D SR-SIM of colocalization of exogenous mitochondria with early endosomes at 4 h. Nuclei were stained with DAPI and both endogenous and exogenous mitochondria were detected with the MTC02 antibody. The combined image (left) and each color (right) are shown; scale bars, 10 µm. FIG. 3C shows representative overlaid microscope images of HCF mitochondria and late endosomes at 1 h; scale bars, 10 µm. FIG. 3D shows representative overlaid microscope images of mitochondria with lysosomes at 4 h; scale bars, 10 µm. In FIGS. 3C and 3D, the arrows indicate examples of exogenous mitochondria associated with each compartment, which also reacted with the anti-mitochondria MTC02 antibody. Nuclei were stained with DAPI and images represent twelve separate volumes analyzed at each time (0.5, 1, 2, and 4 h); Scale bars, 0.5 µm.

The transplanted mitochondria were not pro-arrhythmic as evidenced by serial 12 lead electrocardiogram analysis and optical mapping. There was no ventricular tachycardia, bradycardia, fibrillation, or conduction system defects or repolarization heterogeneity and there are no changes in serial ECG, QRS duration or corrected QT interval associated with mitochondrial transplantation. There were no changes in ventricular wall motion disturbances, left ventricle hypertrophy, valve dysfunction, fibrosis, or pericardial effusion either at the time of injection, or at any time up to 4 weeks following transplantation of autologous mitochondria. Additionally, the transplanted mitochondria produced no immune or inflammatory response. Auto-immune response was measured by anti-mitochondrial antibodies using indirect immunofluorescence was undetectable.

The transplanted mitochondria can act both extra- and intra-cellularly. Once transplanted, mitochondria maintain viability and function for at least 28 days, the temporal limit of the studies conducted to date. The transplanted mitochondria can also immediately increase myocardial ATP levels and ATP synthesis and rapidly alter the myocardial proteome. Proteomic analysis 10 minutes after injection, when improvements in ventricular function were first observed by epicardial echocardiography, showed that protein pathways for the mitochondrion and the generation of precursor metabolites for energy and cellular respiration are significantly higher in treated hearts as compared to control hearts (P < 0.05, Enrichment Score > 2.0). At 10- 60 minutes after transplantation, transplanted mitochondria were internalized into cardiomyocytes by actin-dependent endocytosis. The numbers of mitochondria that were internalized into binucleated cardiomyocytes, were 40.9±11 per nucleus after 1 hour and 218.7 ± 63.7 after 24 hours. Once internalized, the transplanted mitochondria further increase (p<0.05) cellular ATP content and upregulate cardioprotective cytokines (epidermal growth factor, growth-related oncogene, IL-6 and monocyte chemotactic protein-3). These cytokines are associated with enhanced cardiac function by stimulating cell growth, proliferation, and migration, enhancing vascularization, providing protection against cardiomyocyte apoptosis and improving functional cardiac recovery and cardiac remodeling independent of cardiac myocyte regeneration. Ischemia also damages mitochondrial DNA and reduces ATP synthesis, alterations that were associated with poor recovery following cardiac surgery in humans. This Example demonstrated that mitochondrial transplantation replaced, at least partially, damaged mitochondrial DNA with intact mitochondrial DNA and rescued myocardial cell function.

### Example 3: Preliminary Studies in Animals and Humans:

The efficacy of mitochondrial transplantation was validated in a series of *in vitro* and *in vivo* perfused animal heart models. Results from these studies demonstrated that mitochondrial transplantation rescues myocardial cellular viability and significantly enhances post-ischemic myocardial function following ischemia reperfusion injury.

Using ischemial reperfusion protocols in the isolated perfused heart and *in situ* regionally ischemic heart models, it was demonstrated that autologous mitochondrial transplantation is safe and efficacious. The studies demonstrated that both direct and vascular delivery of mitochondria significantly ameliorates ischemialreperfusion injury and enhance myocardial cellular viability and myocardial post-ischemic functional recovery. These effects were evident for at least 28 days -the end point of the in-situ survival experiments.

Pediatric patients with significant IRI require mechanical support with extracorporeal membrane oxygenation (ECMO). The use of ECMO has been shown to be a life-saving technique, but it can lead to complications of bleeding, systemic inflammatory response and infection, thus it can be used only for a limited period of time. If myocardial recovery is not achieved within the duration of ECMO support, the patient ultimately succumbs to the complications of ECMO, which can cause IRI both while and after the patient is cannulated. Studies have shown that patients unable to be weaned from ECMO support within 72 hours is a grave prognostic indicator, with overall survival of <30%. A human pilot study was performed and demonstrated that direct injection of autologous mitochondria significantly improved ventricular function in five pediatric patients in order to enable decannulation from ECMO. None of the patients experienced any adverse effects and there were no complications of arrhythmia, intramyocardial hematoma or inflammatory response. This pilot study was the first clinical application of the novel technique of autologous mitochondrial transplantation to improve cardiac function in patients with IRI.

### Example 4: Administering exogenous mitochondria in an aerosol form to respiratory tract results in localization to (and internalization within) cells lining the respiratory tract

To evaluate if exogenous mitochondria prepared and administered as an aerosol form to respiratory tract results in localization to (and internalization within) cells lining the respiratory tract, an aerosol comprising labeled mitochondria was prepared and administered using a nebulizer system, and the bio-distribution in lung tissue was assessed.

### Methods

### Animal Model

The murine model of lung ischemial reperfusion injury was selected for the experiments. C57BL/6J mice are well characterized and are an established model for use in ALI allowing for comparison of results.

### Bio-distribution

Visualization of the bio-distribution of exogenous mitochondria in the lung was performed in the rat rather than the mouse to enhance visualization and distribution analysis. The mouse model does not provide for sufficient area to detect bio-distribution. In brief, Wistar rats (200 g) was anesthetized with intraperitoneal injection of sodium pentobarbital (100 mg/kg) and maintained on 0.5 % inhaled isoflurane. Oral endotracheal intubation using 20G plastic catheter was performed and the rats were mechanically ventilated with tidal volume 10 mL/kg and respiratory rate 130 - 140 breaths/min. A left thoracotomy was performed in 3rd intercostal space and the pulmonary artery exposed. The bio-distribution of exogenous mitochondria was determined by labelling human cardiac fibroblast mitochondria with ¹⁸F Rhodamine 6-G. The use of human mitochondria in a rat, murine or swine model allows for the differentiation between native mitochondria and transplanted mitochondria based on immune reactivity to a monoclonal anti-human mitochondria antibody.

### Vascular Delivery

The ¹⁸F Rhodamine 6-G labelled human cardiac fibroblast mitochondria were delivered as a bolus in 0.3 mL in respiration buffer (250 mmol/L sucrose, 20 mmol/L K+-HEPES buffer, pH 7.2, 0.5 mmol/L K+-EGTA, pH 8.0) via injection to the left pulmonary artery using a tuberculin syringe with a 40 G needle.

### Nebulizer Delivery

The ¹⁸F Rhodamine 6-G labelled human cardiac fibroblast mitochondria in 90 µL respiration buffer was delivered to the whole lung using a Flexivent nebulizer, over total time of 40s (10s nebulization followed by 1 minute of regular mechanical ventilation, repeated 4 times).

### Measurement of Mitochondria Concentration

The mitochondria were allowed to circulate for 30 minutes, following delivery to allow for washout and then the rats were euthanized by carbon dioxide overdose. Mitochondrial bio-distribution was determined by positron emission tomography (PET) and microcomputed tomography (µCT) using an Albira PET/SPECT/CT Preclinical Imaging System (Bruker, Billerica, MA).

### Quantify Cellular Uptake of Delivered Mitochondria

To confirm the presence of exogenous mitochondria in lung tissue and to identify lung cellular uptake, lung sections were obtained from right and left lungs, fixed with paraffin, and were subject to immuno-histochemical analysis.

### Results

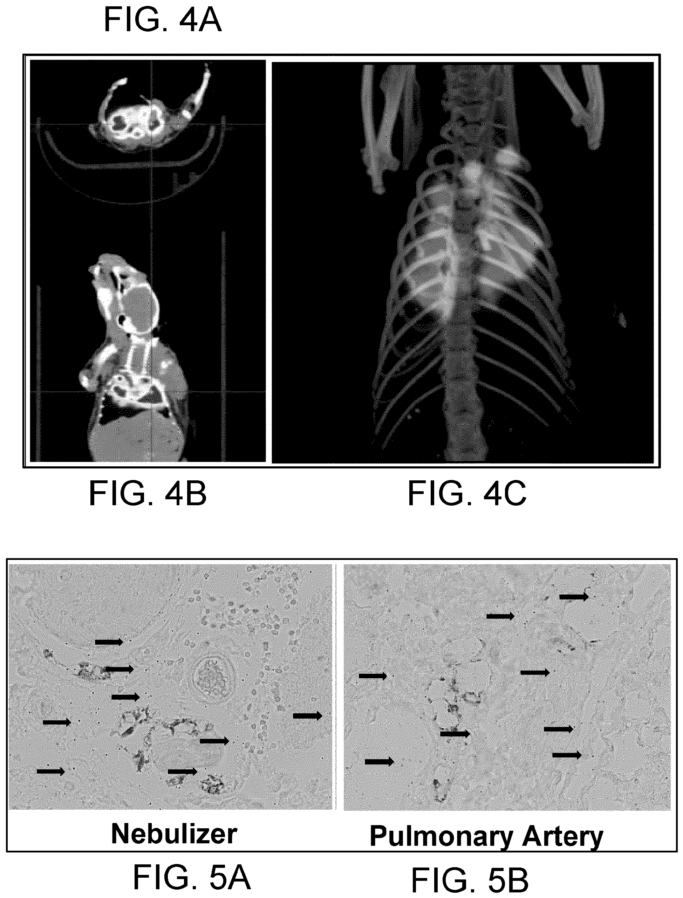

FIGS. 4A-4C show the results of ¹⁸F Rhodamine 6-G mitochondria (6 x 10⁷) delivered to the lungs by vascular infusion through the pulmonary artery in a rat model and imaged using positron emission tomography (PET) and microcomputed tomography (µCT) using an Alvira Imaging System (Bruker, Billerica, MA). These results show that delivery of mitochondria to the lungs results in specific distribution of the transplanted mitochondria throughout the lungs.

FIGS. 5A-5B shows results of immunohistochemical detection and cellular uptake of exogenous mitochondria in mouse lung tissue delivered by a nebulizer and through the pulmonary artery. Human cardiac fibroblast mitochondria were detected using the anti-human mitochondria antibody (MTC02 [biotin] - Abcam, Cambridge, UK, Catalog No.: #ab79479) and Vectastain Elite ABC (Vector Laboratories, Burlingame, CA) and AEC+ substrate chromogen (Dako, Agilent, Santa Clara, CA). The use of human mitochondria in a rat, murine or swine model allows for the differentiation between native mitochondria and transplanted mitochondria based on immune reactivity to a monoclonal anti-human mitochondria antibody. In the figures, mitochondria are shown as black dots. Mitochondria were detected within and around lung endothelial cells, pneumocytes, parenchyma, and alveoli.

### Example 5: Exogenous mitochondria prepared and administered as an aerosol form to respiratory tract can ameliorate symptoms of acute lung ischemia (ALI)

To evaluate if exogenous mitochondria prepared and administered as an aerosol form to respiratory tract of a mouse are sufficient to ameliorate symptoms of acute lung ischemia (ALI) in a mouse ALI model, an aerosol comprising labeled mitochondria was prepared and administered using a nebulizer system, and assays were performed to assess lung function and integrity.

### Methods

### Animal Model

Male C57BL/6J mice (8-10 weeks) Jackson Laboratory, Bar Harbor, ME) were used. All experiments were approved by the Institutional Animal Care and Use Committee at Boston Children's Hospital and conform to the National Institutes of Health guidelines on animal care and use.

### Mitochondrial Isolation

Mitochondria were isolated from skeletal muscle obtained from syngeneic C57BL/6J mice. There is no direct or indirect, acute or chronic alloreactivity, allorecognition or damage-associated molecular pattern molecules (DAMPs) reaction to single or serial injections of syngeneic mitochondria. In brief, skeletal muscle from C57BL/6J mice was dissected and used immediately to isolate syngeneic mitochondria. Mitochondria were isolated, and the number and viability of the isolated mitochondria were determined. The isolated mitochondria were suspended in 0.3 mL respiration buffer and used immediately for mitochondrial transplantation.

### Murine Lung ALI Model:

Surgery was performed, with left hilum occlusion of 2 hours to induce ischemia/reperfusion injury. In brief, C57BL/6J mice were anesthetized with intraperitoneal injection of sodium pentobarbital (100 mg/kg) and maintained during surgery on 0.5 % inhaled isoflurane. Oral endotracheal intubation using 20G plastic catheter was performed and mice were mechanically ventilated with tidal volume 10 mL/kg and respiratory rate 130 - 140 breaths/min. A left thoracotomy was performed in the third intercostal space and the left pulmonary hilum was exposed.

### Acute Lung Ischemia:

The left hilum was occluded at the end of expiration for 2 hours using a microvascular clamp (Roboz Surgical Instrument Co., Gaithersburg, MD, USA) and tidal volume was reduced to 7.5 mL/kg. The surgical incision was covered by gauze soaked in heparin solution containing (60UI of heparin/1ml). At the end of ischemia period, the clip was removed.

### Experimental Groups:

The animals were randomized into three experimental groups:
(a). Vascular Delivery (V): Immediately following the ischemia period and removal of the clip, animals were randomly chosen to receive either 0.3 mL respiration buffer (Vehicle-V) or 0.3 mL respiration buffer containing mitochondria (Mitochondria-V). Vehicle and mitochondria were delivered as a bolus in 0.3 mL via injection to the left pulmonary artery using a tuberculin syringe with a 40 G needle. Mitochondria concentrations were determined by the methods described herein.
(b). Nebulization (N): Immediately following the ischemia period and removal of the clip, animals were randomly chosen to receive either 90 µL respiration buffer (Vehicle-N) or 90 uL respiration buffer containing mitochondria (Mitochondria- N). Vehicle and mitochondria were delivered to the whole lung using a Flexivent nebulizer over total time of 40s (10s nebulization followed by 1 minute of regular mechanical ventilation, repeated 4 times). Mitochondria concentrations were determined. To account for the 2/3 greater volume in nebulizer studies (right lobes and left lobe included), 3 x concentration was used.
(c): Sham control mice underwent thoracotomy without hilar clamping and were mechanically ventilated for 2 hours before returning to the cage. A Sham Control group is required to isolate the specific effects caused by anesthesia, the incisional trauma and potential trauma to the animal tissue during operation.

### Determining optimal mitochondrial concentration required to allow for amelioration of ALI.

2 x 10⁵ autologous mitochondria per gram tissue wet weight provides cardioprotection in the heart. To determine optimal mitochondrial concentration for safety and efficacy in the lung for ALI, several doses were tested, specifically 2 x 10⁵, 2 x 10⁶ and 2 x 10⁷ mitochondria per gram tissue wet weight.

### Lung Functional Analysis

After 24 hours of recovery (reperfusion of the ischemic lung), mice were anesthetized with intraperitoneal injection of sodium pentobarbital (70 mg/kg). A tracheostomy or an endotracheal intubation using plastic 18G catheter was performed and mice were mechanically ventilated. A middle laparotomy and sternotomy was performed to expose the lung and left hilum was clamped to assess the function of the left injured lung. Tidal volume (TV) was reduced to 5 mL/kg and mice were then connected to the specialized rodent ventilator (FlexiVent, SCIREQ, Montreal, Quebec, Canada) to assess Peak Inspiratory Pressure (PIP), Resistance of Respiratory system (Rrs), Compliance of Respiratory System (Crs), Elastance of Respiratory system (Ers) and Tissue Damping (G) of the left lung.

### Damage-Associated Molecular Pattern Molecules (DAMPs) Analysis

To determine if there are any DAMPs related injuries associated with the injection of syngeneic or allogeneic mitochondria, circulating free mitochondrial DNA was detected. Unique primers allowing for the estimation of circulating free mtDNA were used, and the method accounts for nuclear mitochondrial insertion sequences that can affect the accurate quantification of mtDNA. In brief, total DNA (nuclear and mitochondrial) was extracted using a DNeasy Blood & Tissue kit according to manufacturer's instruction (Qiagen, Valencia, CA). DNA was quantified by Nanodrop 2000 (Thermo Scientific, Wilmington, DE) and adjusted to 10 ng/uL. Circulating free mitochondrial DNA (mtDNA) was analyzed by quantitative real-time polymerase chain reaction.

Primers (Eurofins Genomics, Louisville, KY) for mouse mtDNA (mMitoF1, (5' → 3') CTAGAAACCCCGAAACCAAA (SEQ ID NO: 1); mMitoR1 (5' → 3') CCAGCTATCACCAAGCTCGT (SEQ ID NO: 2)), and mouse beta-2 microglobulin (mB2MF1: (5' → 3') ATGGGAAGCCGAACATACTG (SEQ ID NO: 3); mB2MR1: (5' → 3'): CAGTCTCAGTGGGGGTGAAT (SEQ ID NO: 4)) were used to amplify mouse mtDNA. Real-time PCR was performed using the QuantiFast SYBR Green PCR Kit (Qiagen, Valencia, CA). All assays were performed using an Eppendorf Mastercycler ep realplex2 instrument (Eppendorf, Hauppauge, NY). Real time PCR (qPCR) conditions consist of preincubation for 5 min at 95 °C followed by 40 cycles of denaturation at 95 °C for 10 seconds, annealing at 56 °C for 15 seconds and extension at 60 °C for 15 seconds. After the qPCR run was completed, a melting curve analysis were performed consisting of melting at 95 °C for 5 seconds, cooling at 65 °C for 60 seconds, followed by heating to 95 °C with continuous monitoring of fluorescence, and finally cooling at 40 °C for 30 seconds. The mtDNA fold change was normalized to single copy nuclear DNA using the formula, 2-ΔCt (ΔCt=CtMtDNA-CtB2M).

### Imaging Stained Lung Tissue to Assess Lung Integrity

The lungs were collected and divided into 3 equal parts. The upper part of the lung was used to assess tissue edema by calculating wet/dry (w/d) weight ratio. Immediately after the collection, the lung was weighted (wet weight), put in an oven at 70 °C for 72 hours, then weighted again (dry weight) and w/d weight ratio was calculated. The middle part of the lung was immediately fixed in 10% formalin. The tissue was paraffin embedded and sectioned at 5 µm thickness. Serial sections were used for hematoxylin and eosin (H&E) staining, myeloperoxidase staining and TUNEL assay. The basal part of the lung was used for transmission electron microscopy to analyze structural damage in the lung. H&E stained sections were evaluated as a readout of severity of lung injury using a scoring system. Twenty-five random (20X) visual fields per section were analyzed. Lung injury was graded based on infiltration or aggregation of inflammatory cells in air space or vessel wall:
Grade 1: only wall;
Grade 2: few cells (1-5 cells) in air space;
Grade 3: intermediate; and
Grade 4: severe (air space congested).
For interstitial congestion and hyaline membrane formation, the following grades are used:
Grade 1: normal lung;
Grade 2: moderate (25% of lung section);
Grade 3: intermediate (25-50% of lung section); and
Grade 4: severe (50% of lung section);
For hemorrhage, the following grades are used.
Grade 0: absent; and
Grade 1: present.

### Results

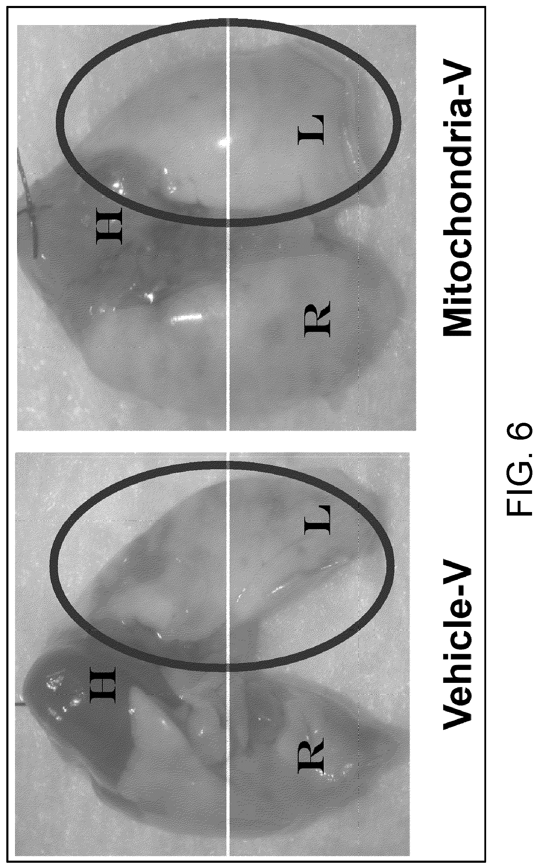

FIG. 6 shows representative macroscopic images of murine lungs following 2 hours left lung ALI ischemia and 24 hours recovery. Lungs that were subject to the ischemia procedure are indicated by the red circle. Right lung is shown as R, the left lung is shown as L. The heart (H) is shown in each image. Vehicle lungs exhibited severe edema and cellular damage (Vehicle-V). In contrast, lungs receiving mitochondria treatment preserved macroscopic cell structure.

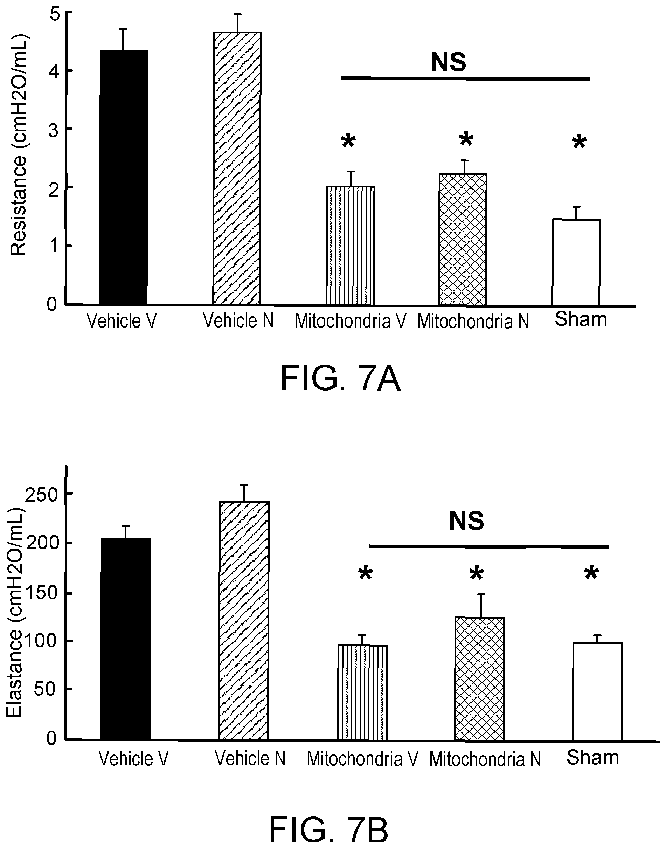

FIGS. 7A-7B show quantified metrics of lung integrity, specifically lung resistance and elastance, in the mouse lung (n=4). Resistance (FIG. 7A) and elastance (FIG. 7B) were measured in mouse lungs subjected to 2 hours left lung ischemia following 24 hours recovery. Vehicle or mitochondria were delivered intravascularly through the left pulmonary artery (V) or by nebulizer (N). The concentration of mitochondria was 2 x 10⁷ mitochondria per gram lung tissue wet weight. Mitochondria delivered by nebulizer were 6 x 10⁷ mitochondria per gram lung tissue wet weight to account for added lung volumes. These results demonstrate that mitochondrial transplantation delivered by vascular or by aerosol using a nebulizer significantly preserves lung resistance and elastance as compared to vehicle treated lungs. Results also show no significant difference between sham controls (no ischemia) and ischemic lungs receiving mitochondria delivered intravascularly through the left pulmonary artery or by a nebulizer. Similar results have been obtained for tissue dampening and lung compliance.

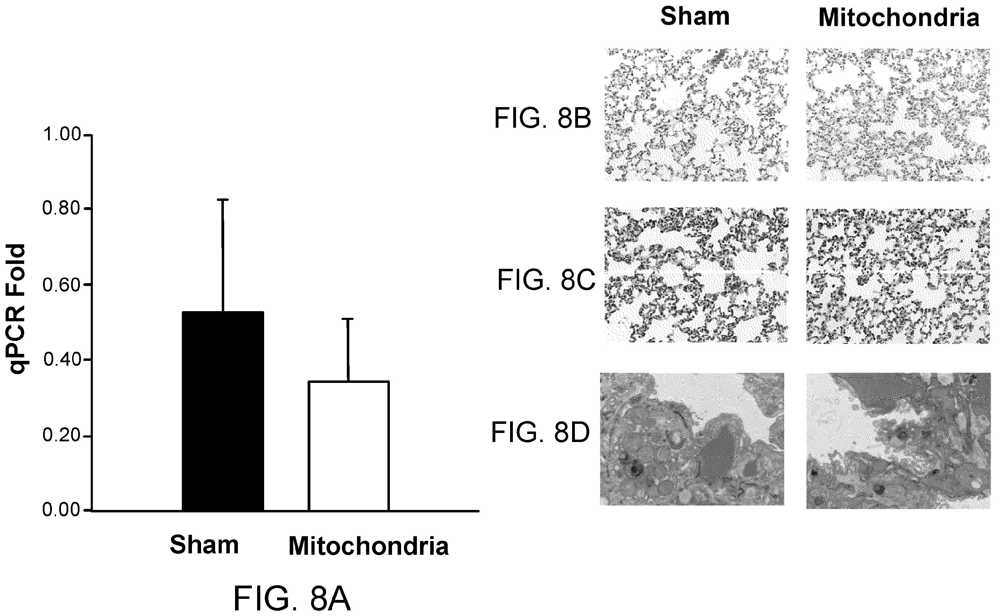

FIG. 8A shows quantification of circulating free mtDNA determined by real-time PCR on whole blood samples in BALB/cJ mice receiving respiration buffer only (Sham) or mitochondria (3 x 10⁷ in the respiration buffer). Blood samples were obtained 10 days after i.p. injection. mtDNA fold change (mean ± SEM) was normalized to single copy nuclear DNA (Beta-2 microglobulin). Results show no increase in circulating free mtDNA with mitochondria injections as compared to Sham (P = 0.96). FIG. 8B shows representative micrographs of hematoxylin and eosin stained lung tissue. FIG. 8C shows representative micrographs of Masson's trichrome stained lung tissue. Lung histology showed no ultrastructural differences in serial lung sections with mitochondria injections as compared to Sham. All sections show normal epithelia from respiratory ducts and alveoli. FIG. 8D shows representative transmission electron micrographs from the inferior right lobe. Electron microscopy images show preserved endothelia and base membranes in all groups. Scale bars are 1 um. Arrows indicate alveolar space. Histological and electron microscopy analysis of lung tissue showed no evidence of inflammation or cellular damage. There was no evidence of myocardial cell necrosis as determined by TTC staining, and no evidence of increased collagen content in myocardial tissue or in lung endothelia or base membrane damage.

### Example 6: Exogenous mitochondria prepared and administered as an aerosol form to respiratory tract are sufficient to ameliorate symptoms in a swine ALI model

The porcine lung has comparable volumes to the human lungs, making it an advantageous model for the study of respiratory function. Porcine lungs have cellular lineages and composition that are comparable with human lungs. The general anatomic distribution of the porcine airways recapitulates that of the human with some minor exceptions. Cartilage surrounding porcine airways is more widespread in distribution than in humans, extending farther down in the tracheobronchial tree. The porcine lung has only 3 generations of bronchioli after the last cartilage plate before reaching the lobule, while humans have approximately 10 generations. Other minor differences include longer porcine terminal bronchioles than in humans as well as shorter and less well-defined porcine respiratory bronchioles. Also similar to humans, swine have a full armamentarium of innate immune effectors in the respiratory tract to protect mucosal surfaces, elicit inflammatory responses, and orchestrate adaptive immune responses. Thus, replicating the experiments in the large animal model (e.g., a swine model) using the same instruments and protocols used in humans can provided better relevance to clinic use in humans.

### Methods

### Animal Model

Yorkshire pigs (male and female, 40-50 kg) are randomly assigned to each group. The pig model has many advantages in terms of clinical relevance. The pig physiology closely resembles the adult human and the model can provide surgical relevance for experimental proof prior to translation to human implementation. The swine lung is an excellent model for the normal human lung, and is an established lung ischemia-reperfusion injury model.

### Experimental Protocol

The pigs are induced with Telazol (4-6mg/kg, IM) and Xylazine (1.1-2.2mg/kg, IM) followed by auricular vein catheter placement. The animal is intubated with a cuffed endotracheal tube and mechanically ventilated via volume-controlled ventilation with TV 10 ml/kg, FiO₂ 0.4, PEEP 5 cm H₂O, RR 12-15 breaths/minute adjusted to obtain PaCO₂ 35-45 mmHg at baseline. General anesthesia is induced with Isoflurane (up to 3% ) and maintained using 0.5-4%).

### Blood Sampling

A femoral artery catheter is placed percutaneously for monitoring mean arterial pressure (MAP) and for arterial blood sampling throughout the procedure. Alternatively, a carotid artery catheter that is placed via a cutdown can be utilized. The femoral vein or jugular vein can be cannulated with a percutaneously placed catheter for CVP monitoring and venous blood sampling. In the case where these vessels cannot be catheterized using ultrasound, a cut down is performed, and a catheter is placed in the vessel under direct visualization. The wound is then sutured with Vicryl or PDS thread (4-0). Heparin sodium 200 UI/kg is given via the auricular vein prior to clamping of left pulmonary hilum to reach a goal ACT > 300 seconds. ACT is checked throughout the surgery, if necessary, the coagulation can be adjusted with additional heparin dose to keep ACT > 300 seconds. If during the surgery ventricular fibrillation occurs, 1% Lidocaine (5 mL, iv) is given to the subject. Lactate ringers solution or saline is infused via the auricular vein or femoral vein throughout the procedure to support arterial blood pressure. Following verification of deep anesthesia, a left thoracotomy is performed over the 5^{th} intercostal space in order to access the left pulmonary hilum. Alternatively, a median sternotomy can be used if access to the hilum is limited in the thoracotomy approach. The pulmonary artery is cannulated with a PVC umbilical catheter for PAP monitoring. Then left atrium is then cannulated via the left atrial appendage using a PVC umbilical catheter to measure LAP and for blood sampling. Core temperature is continually monitored and maintained using a heating pad.

### Blood Flow Monitoring

The ascending aorta and the main pulmonary artery can be dissected and transonic flow probes are attached to monitor blood flow.

### Assessment of Myocardial Function

Myocardial function can be assessed by placing a conductance catheter directly into the RV and LV cavity. The catheters can be inserted through a purse string suture. The purse string suture can prevent bleeding and can secure the catheter in place. Alternatively, the RV catheter can be placed via central venous system via jugular vein. Recordings can be continuously obtained throughout the experiment.

### Echocardiography

Epicardial echocardiography can be performed to determine myocardial function. Epicardial 2D echocardiograms are obtained at the baseline, immediately prior to the injection of autologous mitochondria or respiration buffer and at 3 hours following injection.

### Mitochondrial Isolation

The pectoralis major or rectus abdominis muscle can be located and dissected and two small pieces of muscle tissue (approximately 0.01 g) can be surgically extracted using a biopsy punch and used for mitochondrial isolation under sterile conditions. Mitochondrial isolation can be performed in less than 30 min in keeping with current laboratory standards in order to maintain clinical relevance.

### Left Lung Acute Ischemia

The left main bronchus can be stripped for a length of 1 cm to ablate any bronchial circulation. The pulmonary hilum - artery, veins and bronchus, is clamped for 90 min. The ventilation can be adjusted for single lung ventilation to TV 6 ml/kg, PEEP 5 cmH₂O, FiO₂ is adjusted based on SO₂ levels. Single lung ventilation is maintained for 90min. If during the ischemic period the maximal airway pressure, Pmax increases to as high as 20-22 cmH₂O, the volume-controlled ventilation can be switched to pressure-controlled ventilation with Pmax 20-22 cmH₂0 in order to prevent a barotrauma of the lung. Bronchial clamping can be confirmed visually by deflated left lung with absence of inspiratory and expiratory movements.

### Experimental Groups:

(a) Sham control group - the left hilum can be dissected but not clamped (no left lung ischemia is present) and the animal can be monitored for 90 minutes and then for additional 3 hours during reperfusion. Following 90 minutes of left lung ischemia, the hilar clamp can be released, and the left lung receives either a single 15 mL injection of sterile respiration buffer (Vehicle-V) or a single 15 mL injection of sterile respiration buffer containing mitochondria (Mitochondria-V).
(b) Vascular delivery of mitochondria or vehicle injections can be administered as a bolus to the left pulmonary artery using an 18 G needle. The injection site can be sutured with size 4-0 Prolene suture to control bleeding.
(c) Nebulizer delivery can be performed using a commercial grade nebulizer (Allied Health Care) affixed to the ventilator circuit.

Reperfusion can continue for a period of 3 hours. The animals remain under anesthesia during this time.

### Data collection

MAP, CVP, PAP, LAP will be recorded at the baseline, at the beginning of the reperfusion period, immediately prior to the injection of autologous mitochondria or respiration buffer, and at 1,2,3 hrs. during reperfusion. Respiration measurements for inspiratory peak pressure, inspiratory plateau pressure, and arterial and mixed venous blood for ABG will be performed at the baseline, at the beginning of reperfusion period, immediately prior to the injection of autologous mitochondria or respiration buffer, and at 1, 2, 3 hours during reperfusion.

### Bronchoalveolar lavage

Left lung and right lung lavage can be performed at the end of reperfusion period. Lavage can be performed under bronchoscopy guidance after introducing a catheter through the mouth via the trachea in the caudal lobe first of the left then the right lung. Then 30 ml of saline can be introduced into the lung and the fluid can be aspirated after one minute. Following this, another 15 ml of saline can be introduced into the lung and aspirated after one minute. The aspirated fluid can be taken for the further analysis. The BAL fluid can be immediately centrifuged at 4 °C at 500 x G for 5 minutes, and the supernatant can be collected and stored at -80 °C until further analysis to determine the expression levels of cytokine and chemokines. Cytokine and chemokine analysis can be performed by Eve Technology (Calgary, AB, Canada) The following biomarkers can be tested: Eotaxin, G-CSF, GM-CSF, IFNgamma, IL-1alpha, IL-1beta, IL-2, IL-3, IL-4, IL-5, IL-6, IL-7, IL-9, IL-10, IL-12 (p40), IL-12 (p70), IL-13, IL-15, IL-17A, IP-10, KC, LIF, LIX, MCP-1, M-CSF, MIG, MIP-1alpha, MIP-1beta, MIP-2, RANTES, TNFalpha, VEGF. All samples can be run in triplicates.

### Primary Outcome Measurements

Hemodynamic measurements including e.g., HR, LVPDP, CF, LVEDP, +dP/dT, CO, PVR, LAP, and lung function measurements including e.g., Oxygen -PaO₂, PpvO₂, OI, SO₂, AaDO₂, compliance, elastance, resistance, Cdyn, PEEP, Ccs, PIP, Rrs, Crs, Ers and G (tissue damping of the left lung) and lung pressure, volume loops can be performed at the baseline, the beginning of reperfusion period, immediately prior to the treatment of autologous mitochondria or respiration buffer, and at 1,2,3 hours of reperfusion. Bronchoalveolar lavage of left and right lung is performed at the end of the reperfusion period. Biochemical and proteomic, cytokine, chemokine and immune responses can be determined. Pulmonary inflammatory responses can be examined by analyzing PPI, OI, histology, and expressions of inflammatory cytokines. Lung apoptosis can be determined by TUNEL and lung edema. Lung function pre- and post- ischemia can be determined for each mitochondrial concentration and biochemical and histological analysis can be performed to determine efficacy for each concentration.

### Statistical Plan and Data Analysis

Power analysis indicated that a sample size of 6 animals per group provides over 85% power to detect a difference equal to twice the within group standard deviation (α=0.05, two-sided), and 82% to detect a difference equal to 2.5 times the within group standard deviation (α=0.0083, two-sided, the Bonferroni adjusted significance criterion for post hoc comparisons between 5 groups). For presence/absence of a finding, 6 animals per group would have 95% power to detect an effect occurring in at least 40% of the animals, while 10 animals per group would have 95% power to detect an effect occurring in at least 26% of the animals. A sample of 10 animals can provide 80% statistical power (2-tailed α = 0.05, β = 0.20) to detect a mean difference of 10% or more between the treatment and control arms, assuming a pooled standard deviation of 10% (effect size = 1.0) with an independent groups Student t-test (nQuery Advisor version 7.0, Statistical Solutions, Cork, Ireland). This sample size can also provide 80% power to capture a 24 hour difference between the groups using a Mann-Whitney U-test. power. Based on preliminary studies and animal mortality, typically each experimental group consists of approximately 6 to 10 animals. The mean ± the standard error of the mean for all data is calculated for all variables. Simple pre- vs. post-interventional comparisons are made using a paired two tailed Student's T-test, if the data is normally distributed, or a Wilcoxon Signed Rank test for other continuous data. When there is only a single normally distributed variable for an animal, a one-way analysis of variance is used to compare groups, and extensions in the generalized linear model framework to assess the effects of other variables. Where there are multiple measurements for an animal, mixed model analysis of variance (MM-ANOVA) can be used to assess statistical significance with group and / or drug as fixed effects and incorporate repeated measurements over time as a within subject factor. Generally, autoregressive correlation (AR (1)) correlations can be used to model the relationship between measurements over time. AR (1) is a standard method for modeling data over time and means that measurements close together are more correlated than measurements further apart. For variables which are not normally distributed, data transformations can be performed to normalize the data (e.g., log transformations), or the data can be analyzed using generalized estimating equations.

### Statistical Significance

A P < 0.05 threshold indicates statistical significance. Post hoc comparisons between groups are adjusted for multiple comparisons using a Bonferroni correction. Efficacy can be compared using Fisher's exact test. Randomization can be performed using the R software package based on a 1:1 allocation using a Uniform (0,1) number generator. Statistical analysis can be conducted using SAS version 9.3 (SAS Institute, Cary, NC).

### Results

It is expected that exogenous mitochondria prepared and administered as an aerosol form to respiratory tract are sufficient to ameliorate symptoms in a swine ALI model.

### EXAMPLE 7: Exogenous mitochondria prepared and administered as an aerosol form to respiratory tract are sufficient to ameliorate symptoms in a mouse ALI model

To evaluate if exogenous mitochondria prepared and administered as an aerosol form to respiratory tract of a mouse are sufficient to ameliorate symptoms of acute lung ischemia (ALI) in a mouse ALI model, an aerosol comprising labeled mitochondria was prepared and administered using a nebulizer system, and assays were performed to assess lung function and integrity.

### Methods

### Animals

Male C57BL/6J mice (8-10 weeks, n = 35; Jackson Laboratory, Bar Harbor, ME) were used. All experiments were approved by the Institutional Animal Care and Use Committee at Boston Children's Hospital and conformed to the National Institutes of Health guidelines on animal care and use.

### Mitochondrial isolation

Gastrocnemius muscle from C57BL/6J mice (n = 6) was dissected and used immediately to isolate syngeneic mitochondria. Mitochondria were isolated and the number and viability of the isolated mitochondria was determined as previously described. The isolated mitochondria were suspended in either 0.3 mL respiration buffer (250 mmol/L sucrose, 20 mmol/L K+-HEPES buffer, pH 7.2, 0.5 mmol/L K+-EGTA, pH 8.0) for vascular delivery group or in 90 µL repspiration buffer for nebulization delivery group and used immediately for mitochondrial transplantation.

### Murine lung IRI model

The experimental protocol is shown in Figure 1. Surgery was performed according to the procedure previously described with several modifications. In brief, C57BL/6J mice (n = 29) were anesthetized with intraperitoneal injection of sodium pentobarbital (100 mg/kg) and maintained during surgery on 0.5 % inhaled isoflurane. Oral endotracheal intubation using 20G plastic catheter was performed and mice were mechanically ventilated with tidal volume 10 mL/kg and respiratory rate 130 - 140 breaths/min. Left thoracotomy was performed in 3^{rd} intercostal space and the left pulmonary hilum was exposed. The left hilum was occluded at the end of expiration for 2 hours using a microvascular clamp (Roboz Surgical Instrument Co., Gaithersburg, MD, USA) and tidal volume was reduced to 7.5 mL/kg. The surgical incision was covered by gauze soaked in heparin solution containing (60UI of heparin/1ml). At the end of ischemia period the clip was removed. The animals were randomized into 2 experimental groups: vascular delivery (V) or nebulization (Neb) group. In the vascular delivery group at the beginning of reperfusion either 1 x 10⁸ mitochondria in 0.3 mL respiration buffer (Mito V, n = 6) or 0.3 mL respiration buffer (Vehicle V, n = 7) was delivered to the left lung via injection to the left pulmonary artery using a tuberculin syringe with a 40 G needle. In the delivery via nebulization group at the beginning of reperfusion either 3 x 10⁸ mitochondria in 90 µL respiration buffer (Mito Neb, n = 6) or 90 µL respiration buffer (Vehicle Neb, n = 6) was delivered to the whole lung over total time of 40s (10s nebulization followed by 1 minute of regular mechanical ventilation, repeated 4 times). The mice were allowed to recover and returned to their cage for 24 hours. Sham control mice (Sham, n = 4) underwent thoracotomy without hilar clamping and were mechanically ventilated for 2 hours before returning to the cage.

### Mitochondrial Biodistribution and Uptake

In additional experiments, Wistar rats (200-250 g, n = 6, Charles River Laboratories, Worcester, MA) were used for visualization of mitochondrial uptake in the lung. A donor rat (n = 2) was used for syngeneic mitochondria isolation and ¹⁸F-Rhodamine 6G mitochondrial labeling. Wistar rats (n = 3) were anesthetized and maintained on 2 - 3% inhaled isoflurane. Following, in one set of rats, a sternotomy was performed and the pulmonary trunk was exposed. The ¹⁸F-Rhodamine 6G labeled mitochondria (1 x 10⁹ in 0.5 mL respiration buffer) were delivered to the lungs via injection to the pulmonary trunk using a tuberculin syringe with a 30 G needle.

In another group of Wistar rats (n = 3), ¹⁸F-Rhodamine 6G labeled mitochondria (1 x 10⁹ in 0.3 mL respiration buffer) were delivered to the lungs as an aerosol via nebulization. Ten minutes after delivery of mitochondria, the animals were euthanized in a CO₂ chamber and imaged by positron emission tomography (PET) and microcomputed tomography (µCT).

In a separate set of C57BL/6J mice (n = 4), mitochondria were isolated from human cardiac fibroblasts as previously described²⁹ and delivered to mouse lung tissue via nebulizer and via pulmonary artery. The use of human mitochondria in mouse lungs allowed for differentiation between endogenous mouse mitochondria and transplanted human mitochondria based on immune reactivity to a monoclonal anti-human mitochondrial antibody (biotin-MTCO2, Abcam, Cambridge, MA). Mitochondria were detected using the anti-human mitochondria antibody and visualized using Vectastain (Vector Laboratories, Burlingame, CA) and AEC+ substrate chromogen (Dako, Agilent, Santa Clara, CA) as previously described.

### Lung function

After 24 hours of reperfusion mice were anesthetized with intraperitoneal injection of sodium pentobarbital (70 mg/kg). Tracheostomy and endotracheal intubation using plastic 18G catheter were performed and mice were mechanically ventilated. Next, middle laparotomy and sternotomy were performed to expose the lung and right hilum was clamped to assess the function of the left injured lung. TV was reduced to 5 mL/kg and mice were then connected to the specialized rodent ventilator (FlexiVent, SCIREQ, Montreal, Quebec, Canada) to assess Dynamic Compliance of Respiratory System (Dynamic Compliance), Resistance of Respiratory System (Resistance), Tissue Damping (as an indicator of peripheral tissue resistance), Peak Inspiratory Pressure, and Inspiratory Capacity of the left lung.

### Tissue analysis

After 24 hours of reperfusion left lung was collected and each lung was divided into 3 parts.

The upper part of the left lung was used to assess tissue edema by calculating wet/dry (w/d) weight ratio. Immediately after the collection, the lung was weighted (wet weight), put in an oven at 70 °C for 72 hours, then weighed again (dry weight) and w/d weight ratio was calculated.

The middle part of the left lung was immediately fixed in 10% formalin. The tissue was paraffin embedded and sectioned at 5µm thickness. Serial sections were used for hematoxylin and eosin (H&E) staining, myeloperoxidase staining and TUNEL assay.

H&E stained sections were evaluated for severity of lung injury using previously described scoring system. Five random (15X) visual fields per section were analyzed. Lung injury was graded using following: Grade 1 represented normal pulmonary histology; Grade 2 indicated mild neutrophil leukocyte infiltrations and mild to moderate interstitial congestion; Grade 3 represented moderate neutrophil leukocyte infiltration, perivascular edema formation and partial destruction of pulmonary architecture and Grade 4 included dense neutrophil leukocyte infiltration, abscess formation and complete destruction of pulmonary architecture.

Myeloperoxidase (MPO) staining was performed as previously described and sections were evaluated for neutrophil infiltration. The number of neutrophils was determined in 5 random (15X) visual fields per section.

TUNEL assay was performed as previously described²⁹. The number of TUNEL-positive nuclei was expressed as a percentage of all cells counted in 16 random (10x) visual fields per section.

The basal part of the left lung was used as previously described for transmission electron microscopy (TEM) to analyze structural damage in the lung.

### Bronchoalveolar lavage (BAL) analysis

After lung function assessment, the left lung underwent BAL using 1 mL saline solution. The BAL fluid was immediately centrifuged at 4 °C at 500 x G for 5 minutes, and the supernatant was collected and stored at -80 °C until further analysis to determine the expression levels of cytokine and chemokine by Eve Technology (Calgary, AB, Canada). The following biomarkers were tested: Eotaxin, G-CSF, GM-CSF, IFNgamma, IL-1alpha, IL-1beta, IL-2, IL-3, IL-4, IL-5, IL-6, IL-7, IL-9, IL-10, IL-12 (p40), IL-12 (p70), IL-13, IL-15, IL-17A, IP-10, KC, LIF, LIX, MCP-1, M-CSF, MIG, MIP-1alpha, MIP-1beta, MIP-2, RANTES, TNFalpha, VEGF. All samples were run in triplicates.

### Statistical Analysis

All data were collected by observers blinded to the treatments. All data are expressed as mean ± standard error of the mean (SEM). Statistical analysis was performed by non-parametric Mann-Whitney U test for all data except BAL analysis results, where between-group comparisons were evaluated with one-way ANOVA. Statistical significance was defined by an exact two-tailed P < 0.05.

### Results

### Lung uptake of radiolabeled mitochondria

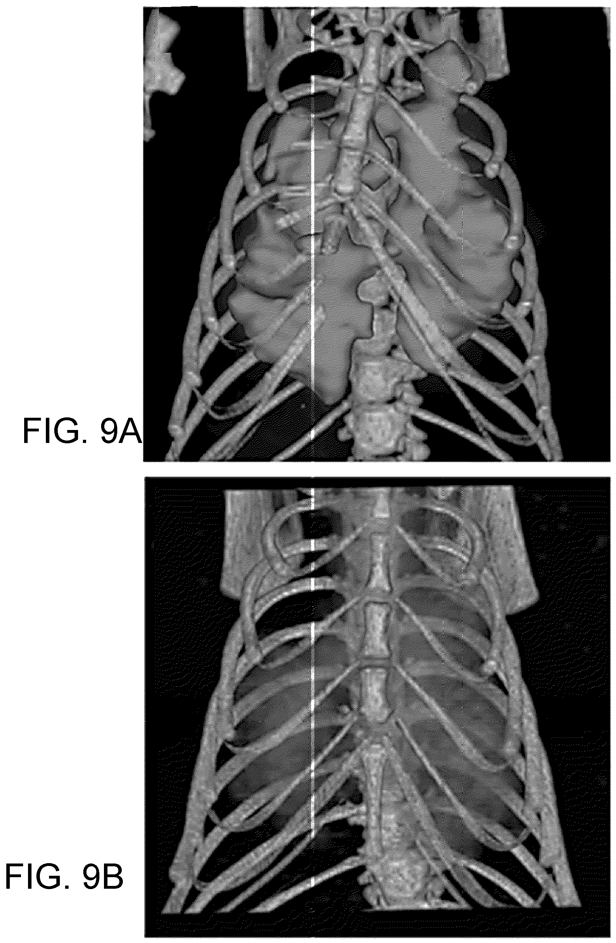

¹⁸F-Rhodamine 6G radiolabeled mitochondria delivered either via vascular delivery **(****FIG. 9A****)** or via nebulization **(****FIG. 9B****)** were taken up diffusely by the lungs and were not present in any other region of the body.

### Lung tissue uptake of human mitochondria

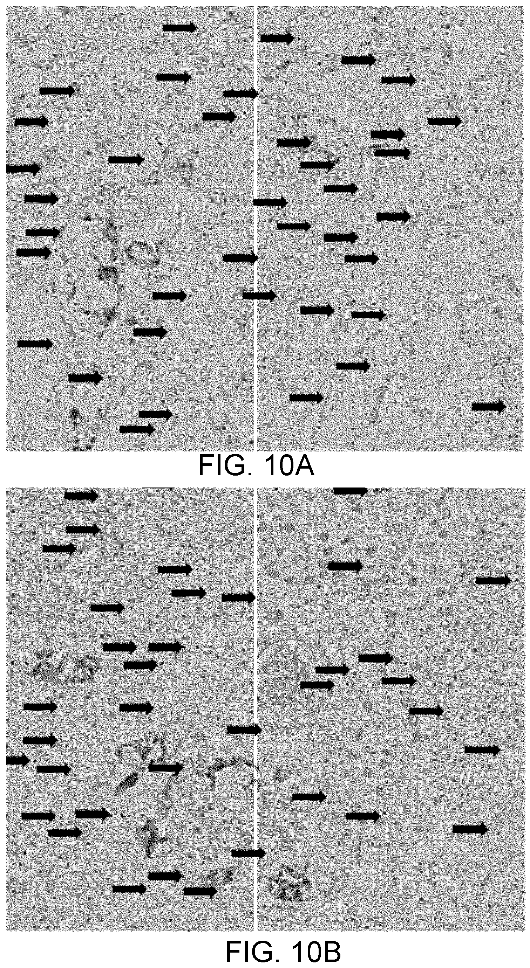

Exogenous mitochondria from human cardiac fibroblast, delivered either via vascular delivery **(****FIG. 10A****)** or via nebulization **(****FIG. 10B****),** were detected within and around lung endothelial cells, pneumocytes, parenchyma, and alveoli.

### Lung functional performance

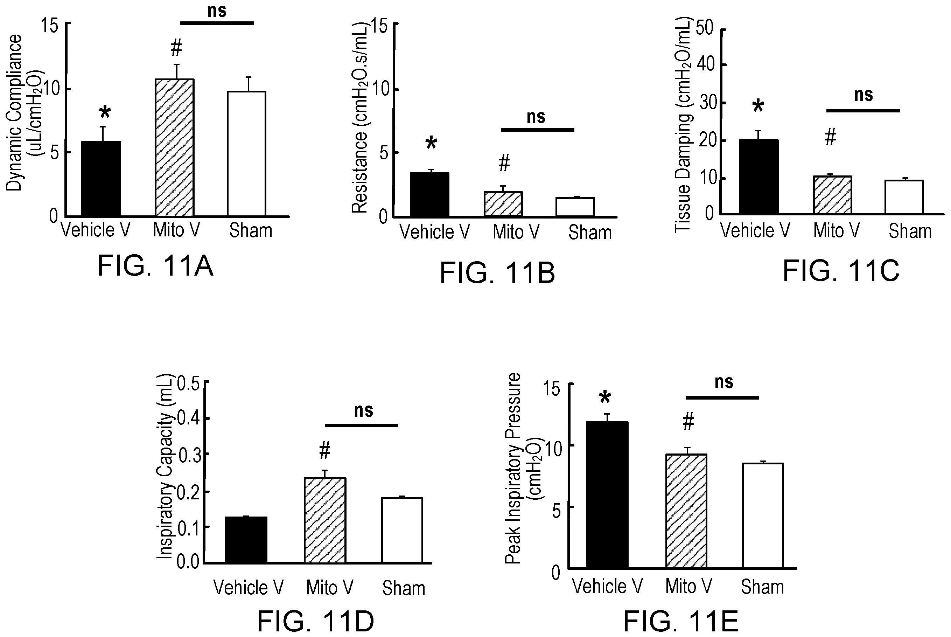

After 2 hours of ischemia and 24 hours of reperfusion, the mice that received Mitochondria via vascular delivery had significantly increased Dynamic Compliance (10.79 ± 1.11 µL/cmH₂O) and Inspiratory Capacity (0.24 ± 0.02 mL) and significantly decreased Resistance (2.02 ± 0.28 cmH₂O), Tissue Damping (10.49 ± 0.91 cmH₂O/mL) and Peak Inspiratory Pressure (9.48 ± 0.40 cmH₂O) and as compared to Vehicle V (5.91 ± 0.46, 0.127 ± 0.01, 3.33 ± 0.40, 20.02 ± 2.75 and 11.96 ± 0.74 respectively, P < 0.05 each; **FIG. 11A-E).** All lung functional parameters in Mito V group were not significantly different as compared to Sham lungs (9.75 ± 0.35, 0.18 ± 0.01, 1.51 ± 0.20, 9.46 ± 0.63 and 8.62 ± 0.20 respectively, P > 0.05 each; **FIG. 11A-E).**All parameters in Vehicle V lungs were significantly different (P < 0.05) as compared to Sham lungs, except for Inspiratory Capacity (P > 0.05).

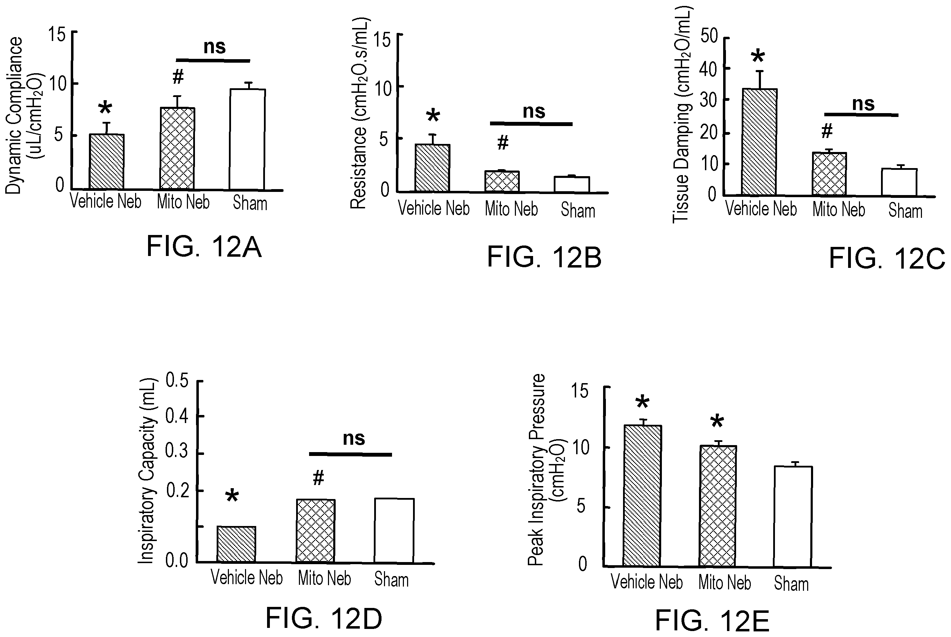

After 2 hours of ischemia and 24 hours of reperfusion, the mice that received mitochondria via nebulization, had significantly increased Dynamic Compliance (7.88 ± 1.05 uL/cmH₂O) and Inspiratory Capacity (0.18 ± 0.02 mL) and significantly decreased Resistance (2.13 ± 0.23 cmH₂O), Tissue Damping (13.63 ± 1.55 cmH₂O/mL) and Peak Inspiratory Pressure (10.18 ± 0.40 cmH₂O) and as compared to Vehicle Neb (5.13 ± 0.93, 0.10 ± 0.02, 4.74 ± 0.83, 34.02 ± 5.04 and 11.82 ± 0.53 respectively, P < 0.05 each; **FIG. 12A-D).**

All lung functional parameters in Mito Neb group were not significantly different as compared to Sham lungs (9.75 ± 0.35, 0.18 ± 0.01, 1.51 ± 0.20 and 9.46 ± 0.63 respectively, P > 0.05 each; Figure 3D-F, I, J) except Peak Inspiratory Pressure (8.62 ± 0.20, P < 0.05). All parameters in Vehicle Neb lungs were significantly different (P < 0.05) as compared to Sham lungs.

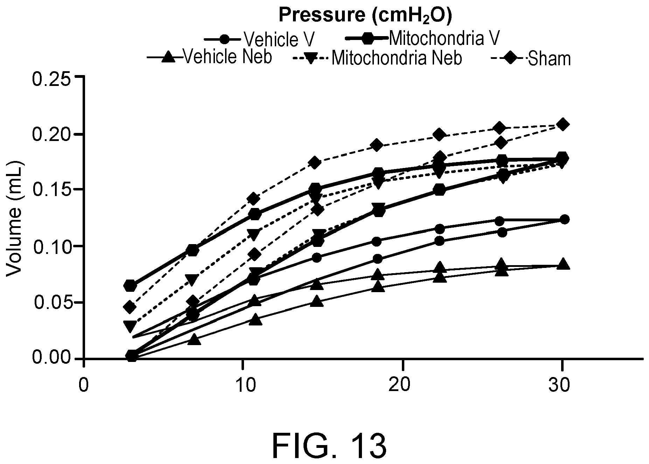

Representative pressure-volume loops demonstrated that Vehicle V and Vehicle Neb had a lower lung volume capacity and higher pressures as compared to Mito V, Mito Neb and Sham groups **(****FIG. 13****).**

### Lung tissue injury

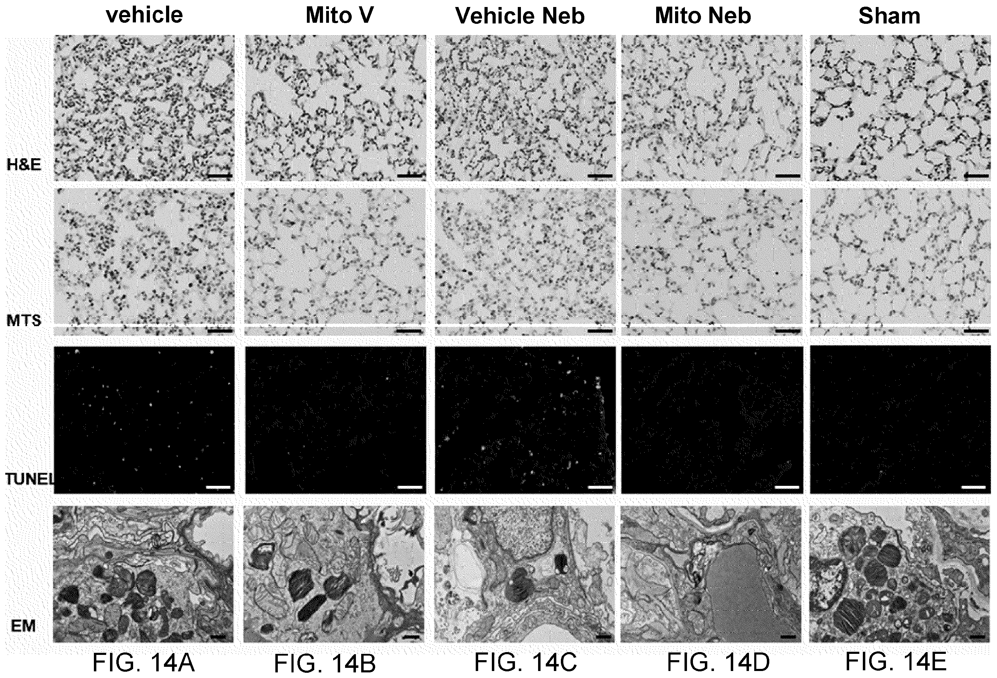

H&E analysis showed significantly decreased inflammatory cells infiltration and interstitial congestion with no signs of destruction of lung architecture in Mito V and Mito Neb lungs as compared to Vehicle V and Vehicle Neb, respectively (grade 1.8 ± 0.1, grade 2.5 ± 0.1; respectively for vascular delivery group; grade 1.7 ± 0.1, grade 2.5 ± 0.1; respectively for nebulization group, P < 0.05, both; **FIG. 14****,** **FIG. 15A-E).** Significantly improved preservation of the lung tissue was observed in Sham lungs as compared to all groups (grade 1.3 ± 0.1, P < 0.05).

Significantly decreased count of neutrophils was observed in Mito V and Mito Neb groups as compared to Vehicle V and Vehicle Neb, respectively (120.2 ± 5.5, 159.9 ± 6.5; respectively for vascular delivery group; 77.8 ± 3.8, 125.7 ± 4.9; respectively for nebulization group, P < 0.05, both, **FIG. 14****,** **FIG. 15B, FIG. 15E****).** Neutrophil count was significantly reduced in Sham lungs (55.3 ± 3.6, P < 0.05) as compared to Mito V, Vehicle V and Vehicle Neb lungs. There was no significant difference observed in number of neutrophils in Sham group as compared to Mito Neb (P > 0.05).

TUNEL analysis showed significantly decreased count of apoptosis positive nuclei observed in Mito V and Mito Neb groups as compared to Vehicle V and Vehicle Neb, respectively (1.38 ± 0.25, 1.55 ± 0.18; respectively for vascular delivery group; 3.1 ± 0.38, 3.15 ± 0.25; respectively for nebulization group, P < 0.05, both). There was no significant difference in apoptosis positive nuclei count in Sham lungs (0.3 ± 0.1, P < 0.05) as compared to Mito V and Mito Neb groups **(****FIG. 14****,** **FIG. 15C, FIG. 15F****).**

Following 24 hours of reperfusion, TEM from the inferior left lobe showed preserved endothelia and base membranes in Vehicle V and Neb and Mito V and Neb. There is no difference in alveolar space. This indicates there is no base membrane damage (Figure 4).

There was no significant difference found in wet-to-dry weight percentages between Vehicle V left (88.9 ± 0.5%) and right lung (87.1 ± 1.1%), Mito V left (88.5 ± 0.3%) and right lung (88.3 ± 0.6%), Vehicle Neb left (87.5 ± 1.1%) and right lung (89.2 ± 1.1%) and Mito Neb left (88.5 ± 1.2%) and right lung (89.3 ± 0.6%) and Sham left (88.7 ± 1.0%) and right lung (90.1 ± 1.0%; P > 0.05 each; **FIG. 15G****).**

### Bronchoalveolar lavage cytokines

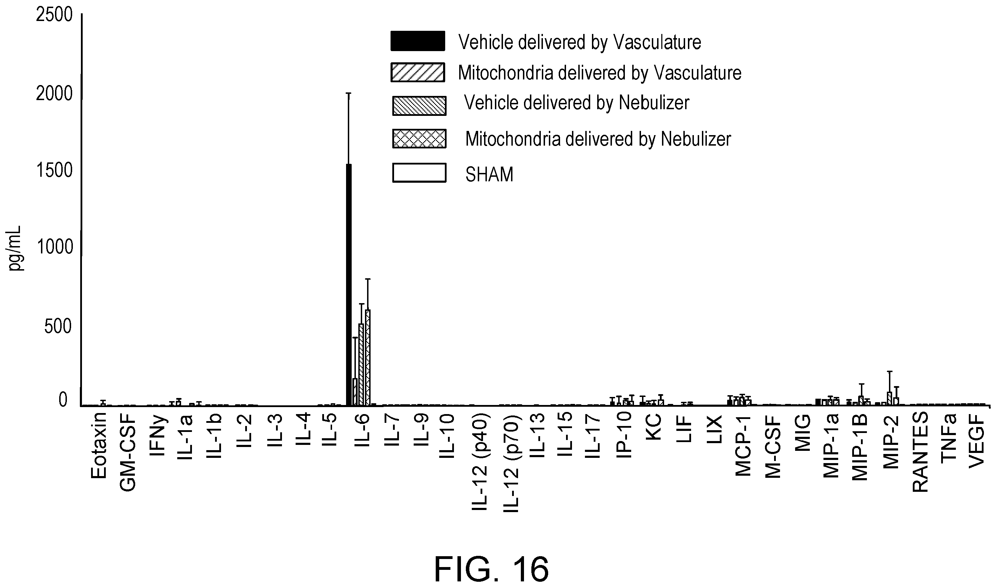

At 24 hours of reperfusion, there were no significant differences in cytokines and chemokines between groups **(****FIG. 16****).**

### Discussion

Mitochondrial damage, consisting of reactive oxygen species-induced toxicity, failure of cellular bio-energetics, upregulated apoptosis, cytokine production, and innate immunity, during IRI plays a significant role in acute lung injury. In this study, we have demonstrated the efficacy and safety of mitochondrial transplantation in a murine model of lung IRI. After 24 hours of reperfusion following ischemic injury, mitochondrial transplantation improves lung functional performance and decreases lung tissue injury.

Previous studies investigating murine lung IRI have utilized 60 min to 90 mins of ischemia time. These experiments provided detailed analysis of lung tissue damage. However, they either did not provide any functional analysis or lung function was measured in non-physiologic, isolated ex vivo system. In our study FlexiVent machine was used in order to achieve in vivo functional measurements. Based on our preliminary experiments we chose 2 hours of ischemia time. Shorter ischemia times caused insufficient lung IRI and would not allow for functional evaluation and comparative analysis between our study groups. ¹⁸F-Rhodamine 6G radiolabeled mitochondria were used to demonstrate distribution of delivered mitochondria. We delivered radiolabeled mitochondria either via vascular delivery or nebulization. Our results show mitochondrial distribution throughout the lungs in both groups. The mitochondria delivered as an aerosol via nebulization were found along trachea and bronchial tree suggesting that mitochondria adhere to the airway tissue already during the first contact.

In our studies, human mitochondria were used to determine cellular uptake of transplanted mitochondria. Human mitochondria allow for targeted labeling of transplanted mitochondria and therefore exclude the possibility of labeling the endogenous murine mitochondria. The human mitochondria were found in all cells throughout the lungs, demonstrating that mitochondria delivered via either vasculature or nebulization were effectively transplanted.

Two different mitochondrial delivery methods were used in this study. The mitochondria can be delivered to the targeted organ via vasculature. To allow for less invasive, but still clinically relevant and easily utilized method, delivery via nebulization was used as an additional study group. After 24 hours of reperfusion, significant functional improvement was observed in lungs receiving mitochondria both via vascular delivery and nebulization when compared to vehicle lungs. In one functional parameter, PIP, there was significant difference observed between Mito V vs Vehicle V, but no significant difference was observed when comparing Mito Neb vs Vehicle Neb. This could imply an increased effectiveness of mitochondria delivered via vasculature. However, this observation could be explained by the fact that mitochondria delivered via nebulization are not delivered as far as mitochondria delivered via vasculature, and it would have been preferred to deliver a higher concentration of mitochondria because of their uptake to the trachea and bronchial tree as well. Experiments can be performed to determine the optimal mitochondrial concentration delivered by nebulization are necessary. It is also important to note, that tissue analysis showed significant improvement of cell viability after 24 hours of reperfusion in both Mito V and Mito Neb when compared to their Vehicle groups.

In conclusion, we have demonstrated the efficacy and safety of mitochondrial transplantation in a murine model of lung IRI. After 24 hours of reperfusion following ischemic injury, mitochondrial transplantation improves lung function and decreases lung tissue injury. These results suggest that this therapy can be used to protect lung tissue from IRI, thus reducing morbidity and mortality in several clinical scenarios such as: lung transplantation, cardiopulmonary resuscitation, pulmonary embolism, sepsis and hypotensive events and procedures during cardiopulmonary bypass or mechanical circulatory support.

## Claims

1. A composition comprising a therapeutically effective amount of mitochondria for use in a method of treating a subject having a respiratory disorder, the method comprising administering said composition to the subject through respiratory tract, wherein the composition is an aerosolized composition, and wherein the composition is converted into an aerosol form prior to administration to the subject by using a nebulizer, a vaporizer, a nasal sprayer, a pressurized metered dose inhaler, or a breath activated pressurized metered dose inhaler.

2. The composition for use of claim 1, wherein the composition comprises respiration-competent mitochondria.

3. The composition for use of claim 1, wherein:
the subject has respiratory failure, reduced respiratory function, lung inflammation, lung carcinoma, skin wrinkles, baldness, and/or cancer, optionally wherein the subject has acute lung injury;
the subject has ischemia, perfusion injury, pulmonary hypoplasia, congenital diaphragmatic hernia (CDH), Poland syndrome, Chest wall disease, pneumonectomy, bronchopulmonary dysplasia, lung injury, inhalation injury, asthma, cystic fibrosis;
the subject has acute respiratory distress syndrome (ARDS), acute lung injury (ALI), respiratory failure, reduced respiratory function, and/or lung inflammation; and
the subject has pneumonia.

4. The composition for use of claim 1, wherein the concentration of mitochondria in the composition is about 1x10⁵ to 5x10⁸ ml⁻¹ or about 1x10⁵ to 1x10⁹ of mitochondria per dose.

5. The composition for use of claim 1, wherein the composition further comprises a therapeutic agent or a diagnostic agent, wherein the therapeutic agent or the diagnostic agent is linked to the mitochondria by a covalent bond, is embedded in the mitochondria, or is internalized within the mitochondria, optionally wherein the therapeutic agent or the diagnostic agent is a therapeutic diagnostic agent, or optionally wherein the therapeutic agent or the diagnostic agent comprises an antibody or an antigen binding fragment thereof, or optionally wherein the therapeutic agent or the diagnostic agent is linked to the mitochondria by a covalent bond, or optionally wherein the therapeutic agent or the diagnostic agent is embedded in or internalized within the mitochondria.

6. The composition for use of claim 1, wherein the mitochondria are genetically modified, or wherein the mitochondria comprise exogenous polypeptides, or wherein the mitochondria comprise exogenous polynucleotides, DNA, RNA, mRNA, micro RNAs, nuclear RNAs, or siRNA.

7. An aerosolized composition comprising
a plurality of liquid droplets comprising a buffer, wherein at least one liquid droplet in the plurality of liquid droplets comprises:
at least one mitochondrion.

8. The aerosolized composition of claim 7, wherein the composition further comprises a therapeutic agent or a diagnostic agent, wherein the therapeutic agent or the diagnostic agent is linked to the mitochondrion by a covalent bond, is embedded in the mitochondrion, or is internalized within the mitochondrion, optionally wherein the mitochondrial agent is selected from the group consisting of a therapeutic agent, a chemotherapeutic agent, or a diagnostic agent, or optionally wherein the mitochondrial agent comprises an antibody or an antigen binding fragment, or optionally wherein the mitochondrial agent is linked to the mitochondrion by a covalent bond, or optionally wherein the mitochondrial agent is embedded in or internalized within the mitochondrion.

9. A device for delivering mitochondria to a subject through respiratory tract, the device comprising:
a housing;
a reservoir disposed within the housing for a composition comprising a therapeutically effective amount of mitochondria, wherein the composition is an aerosolized composition;
an aerosol generator to produce an aerosol form of the composition; and
an outlet through which the composition is delivered to the respiratory tract of the subject, wherein the device is a nebulizer, a nasal sprayer, or an inhaler.

10. The device of claim 9, wherein the aerosol generator is an ultrasonic nebulizer.

11. The device of claim 9, wherein the aerosol generator is a vibrating mesh device.

12. The device of claim 9, wherein the aerosol generator comprises an air compressor, wherein the air compressor cause compressed air to flow through the composition and turn the composition into an aerosol form.

13. A device for delivering mitochondria to a subject through respiratory tract, the device comprising:
a housing;
a composition comprising a therapeutically effective amount of mitochondria disposed within the housing, wherein the composition is an aerosolized composition; and
an outlet configured to deliver the composition from the housing to the respiratory tract of the subject and to aerosolize the composition as the composition passes therethrough.

## Patentansprüche

1. Zusammensetzung, umfassend eine therapeutisch wirksame Menge an Mitochondrien, zur Verwendung in einem Verfahren zur Behandlung eines Individuums mit einer Atemwegserkrankung, wobei das Verfahren Verabreichen der Zusammensetzung an das Individuum über die Atemwege umfasst, wobei es sich bei der Zusammensetzung um eine aerosolisierte Zusammensetzung handelt und wobei die Zusammensetzung vor der Verabreichung an das Individuum unter Verwendung eines Verneblers, eines Verdampfers, eines Nasensprayers, eines unter Druck stehenden Dosierinhalators oder eines atemaktivierten unter Druck stehenden Dosierinhalators in ein Aerosol überführt wird.

2. Zusammensetzung zur Verwendung nach Anspruch 1, wobei die Zusammensetzung respirationskompetente Mitochondrien umfasst.

3. Zusammensetzung zur Verwendung nach Anspruch 1, wobei:
das Individuum an Atemversagen, verminderter Atemfunktion, Lungenentzündung, Lungenkarzinom, Hautfalten, Kahlheit und/oder Krebs leidet, gegebenenfalls wobei das Individuum eine akute Lungenschädigung aufweist;
das Individuum an Ischämie, Perfusionsschaden, Lungenhypoplasie, angeborener Zwerchfellhernie (Congenital Diaphragmatic Hernia, CDH), Poland-Syndrom, Brustwanderkrankung, Pneumonektomie, bronchopulmonaler Dysplasie, Lungenschädigung, Inhalationstrauma, Asthma, Mukoviszidose leidet;
das Individuum an akutem Atemnotsyndrom (ARDS), akuter Lungenschädigung (ALI), Atemversagen, verminderter Atemfunktion und/oder Lungenentzündung leidet; und
das Individuum an Pneumonie leidet.

4. Zusammensetzung zur Verwendung nach Anspruch 1, wobei die Konzentration an Mitochondrien in der Zusammensetzung etwa 1x10⁵ bis 5x10⁸ ml⁻¹ oder etwa 1x10⁵ bis 1x10⁹ Mitochondrien pro Dosis beträgt.

5. Zusammensetzung zur Verwendung nach Anspruch 1, wobei die Zusammensetzung ferner ein therapeutisches Mittel oder ein diagnostisches Mittel umfasst, wobei das therapeutische Mittel oder das diagnostische Mittel über eine kovalente Bindung mit den Mitochondrien verknüpft ist, in die Mitochondrien eingebettet ist oder in den Mitochondrien internalisiert ist, wobei das therapeutische Mittel oder das diagnostische Mittel gegebenenfalls ein therapeutisches diagnostisches Mittel ist oder wobei das therapeutische Mittel oder das diagnostische Mittel gegebenenfalls einen Antikörper oder ein antigenbindendes Fragment davon umfasst oder wobei das therapeutische Mittel oder das diagnostische Mittel gegebenenfalls über eine kovalente Bindung mit den Mitochondrien verknüpft ist oder wobei das therapeutische Mittel oder das diagnostische Mittel gegebenenfalls in die Mitochondrien eingebettet oder darin internalisiert ist.

6. Zusammensetzung zur Verwendung nach Anspruch 1, wobei die Mitochondrien gentechnisch verändert sind oder wobei die Mitochondrien exogene Polypeptide umfassen oder wobei die Mitochondrien exogene Polynukleotide, DNA, RNA, mRNA, Mikro-RNAs, nukleäre RNAs oder siRNA umfassen.

7. Aerosolisierte Zusammensetzung, umfassend
mehrere Flüssigkeitströpfchen, die einen Puffer umfassen, wobei mindestens ein Flüssigkeitströpfchen in den mehreren Flüssigkeitströpfchen
mindestens ein Mitochondrion umfasst.

8. Aerosolisierte Zusammensetzung nach Anspruch 7, wobei die Zusammensetzung ferner ein therapeutisches Mittel oder ein diagnostisches Mittel umfasst, wobei das therapeutische Mittel oder das diagnostische Mittel über eine kovalente Bindung mit dem Mitochondrium verknüpft ist, in das Mitochondrium eingebettet ist oder innerhalb des Mitochondriums internalisiert ist, wobei das mitochondriale Mittel gegebenenfalls aus der aus einem therapeutischen Mittel, einem chemotherapeutischen Mittel und einem diagnostischen Mittel bestehenden Gruppe ausgewählt ist oder wobei das mitochondriale Mittel gegebenenfalls einen Antikörper oder ein antigenbindendes Fragment umfasst oder wobei das mitochondriale Mittel gegebenenfalls über eine kovalente Bindung mit dem Mitochondrium verknüpft ist oder wobei das mitochondriale Mittel gegebenenfalls in das Mitochondrium eingebettet oder darin internalisiert ist.

9. Vorrichtung zur Zuführung von Mitochondrien an ein Individuum über die Atemwege, wobei die Vorrichtung Folgendes umfasst:
ein Gehäuse;
ein Reservoir, das innerhalb des Gehäuses angeordnet ist, für eine Zusammensetzung, die eine therapeutisch wirksame Menge an Mitochondrien umfasst, wobei es sich bei der Zusammensetzung um eine aerosolisierte Zusammensetzung handelt;
einen Aerosolerzeuger zum Herstellen einer Aerosolform der Zusammensetzung; und
einen Auslass, durch den die Zusammensetzung den Atemwegen des Individuums zugeführt wird, wobei es sich bei der Vorrichtung um einen Vernebler, einen Nasensprayer oder einen Inhalator handelt.

10. Vorrichtung nach Anspruch 9, wobei es sich bei dem Aerosolerzeuger um einen Ultraschallvernebler handelt.

11. Vorrichtung nach Anspruch 9, wobei es sich bei dem Aerosolerzeuger um eine Vibrating-Mesh-Vorrichtung handelt.

12. Vorrichtung nach Anspruch 9, wobei der Aerosolerzeuger einen Luftverdichter umfasst, wobei der Luftverdichter bewirkt, dass Druckluft durch die Zusammensetzung strömt und die Zusammensetzung in eine Aerosolform überführt.

13. Vorrichtung zur Zuführung von Mitochondrien an ein Individuum über die Atemwege, wobei die Vorrichtung Folgendes umfasst:
ein Gehäuse;
eine Zusammensetzung, die eine therapeutisch wirksame Menge an Mitochondrien umfasst, die innerhalb des Gehäuses angeordnet ist, wobei es sich bei der Zusammensetzung um eine aerosolisierte Zusammensetzung handelt; und
einen Auslass, der so ausgelegt ist, dass durch ihn die Zusammensetzung aus dem Gehäuse den Atemwegen des Individuums zugeführt und beim Hindurchströmen aerosolisiert wird.

## Revendications

1. Composition comprenant une quantité thérapeutiquement efficace de mitochondries pour une utilisation dans une méthode de traitement d'un sujet atteint d'un trouble respiratoire, la méthode comprenant l'administration de ladite composition au sujet par les voies respiratoires, la composition étant une composition aérosolisée, et la composition étant convertie en une forme d'aérosol avant administration au sujet au moyen d'un nébuliseur, d'un vaporisateur, d'un pulvérisateur nasal, d'un inhalateur-doseur pressurisé ou d'un inhalateur-doseur pressurisé activé par la respiration.

2. Composition selon la revendication 1, la composition comprenant des mitochondries aptes à la respiration.

3. Composition selon la revendication 1, **caractérisée en ce que** :
le sujet présente une insuffisance respiratoire, une fonction respiratoire réduite, une inflammation pulmonaire, un carcinome pulmonaire, des rides cutanées, une calvitie, et/ou un cancer, facultativement, le sujet présentant une lésion pulmonaire aiguë ;
le sujet présente une ischémie, une lésion de perfusion, une hypoplasie pulmonaire, une hernie diaphragmatique congénitale (CDH), le syndrome de Poland, une maladie de la paroi thoracique, une pneumonectomie, une dysplasie bronchopulmonaire, une lésion pulmonaire, une lésion par inhalation, l'asthme, la mucoviscidose ;
le sujet présente un syndrome de détresse respiratoire aigu (SDRA), une lésion pulmonaire aiguë (ALI), une insuffisance respiratoire, une fonction respiratoire réduite et/ou une inflammation pulmonaire ; et
le sujet est atteint d'une pneumonie.

4. Composition selon la revendication 1, dans laquelle la concentration de mitochondries dans la composition est d'environ 1x10⁵ à 5x10⁸ ml⁻¹ ou d'environ 1x10⁵ à 1x10⁹ mitochondries par dose.

5. Composition selon la revendication 1, la composition comprenant en outre un agent thérapeutique ou un agent diagnostique, l'agent thérapeutique ou l'agent diagnostique étant lié aux mitochondries par une liaison covalente, étant incorporé dans les mitochondries ou étant internalisé dans les mitochondries, facultativement, l'agent thérapeutique ou l'agent diagnostique étant un agent diagnostique thérapeutique ou facultativement, l'agent diagnostique comprenant un anticorps ou un fragment de liaison d'antigène de celui-ci, ou facultativement, l'agent thérapeutique ou l'agent diagnostique étant lié aux mitochondries par une liaison covalente ou facultativement, l'agent thérapeutique ou l'agent diagnostique étant incorporé ou internalisé dans les mitochondries.

6. Composition selon la revendication 1, dans laquelle les mitochondries sont génétiquement modifiées, ou dans laquelle les mitochondries comprennent des polypeptides exogènes, ou dans laquelle les mitochondries comprennent des polynucléotides exogènes, de l'ADN, de l'ARN, de l'ARNm, des microARN, des ARN nucléaires ou un ARNsi.

7. Composition aérosolisée comprenant
une pluralité de gouttelettes de liquide comprenant un tampon, au moins une gouttelette de liquide dans la pluralité de gouttelettes de liquide comprenant :
au moins une mitochondrie.

8. Composition aérosolisée selon la revendication 7, la composition comprenant en outre un agent thérapeutique ou un agent diagnostique, l'agent thérapeutique ou l'agent diagnostique étant lié à la mitochondrie par une liaison covalente, étant incorporé dans la mitochondrie ou étant internalisé dans la mitochondrie, facultativement, l'agent mitochondrial étant choisi dans le groupe constitué d'un agent thérapeutique, d'un agent chimiothérapeutique ou d'un agent diagnostique, ou facultativement, l'agent mitochondrial comprenant un anticorps ou un fragment de liaison d'antigène, ou facultativement, l'agent mitochondrial étant lié à la mitochondrie par une liaison covalente, ou facultativement, l'agent mitochondrial étant incorporé ou internalisé dans la mitochondrie.

9. Dispositif d'administration de mitochondries à un sujet par les voies respiratoires, le dispositif comprenant :
un boîtier ;
un réservoir disposé à l'intérieur du boîtier pour une composition comprenant une quantité thérapeutiquement efficace de mitochondries, la composition étant une composition aérosolisée ;
un générateur d'aérosol pour produire une forme d'aérosol de la composition ; et
une sortie par l'intermédiaire de laquelle la composition est administrée aux voies respiratoires du sujet, le dispositif étant un nébuliseur, un pulvérisateur nasal ou un inhalateur.

10. Dispositif selon la revendication 9, dans lequel le générateur d'aérosol est un nébuliseur à ultrasons.

11. Dispositif selon la revendication 9, dans lequel le générateur d'aérosol est un dispositif à mailles vibrantes.

12. Dispositif selon la revendication 9, dans lequel le générateur d'aérosol comprend un compresseur d'air, le compresseur d'air faisant circuler de l'air comprimé à travers la composition et transformant la composition en une forme d'aérosol.

13. Dispositif d'administration de mitochondries à un sujet par les voies respiratoires, le dispositif comprenant :
un boîtier ;
une composition comprenant une quantité thérapeutiquement efficace de mitochondries disposées à l'intérieur du boîtier, la composition étant une composition aérosolisée ; et
un orifice de sortie conçu pour administrer la composition depuis le boîtier aux voies respiratoires du sujet et pour aérosoliser la composition lorsque la composition traverse celui-ci.
